# EUROPEAN PATENT APPLICATION

(11) **EP 4 803 102 A1**
(43) Date of publication of application: **09.09.2026**
(21) Application number: 25162266.8
(22) Date of filing: 07.03.2025
(51) Int. Cl.: A61K 47/60

(54) **TISSUE SELECTIVE IL-22 AGONISTS COMPRISING SPECIFIC AMINO ACID MODIFICATIONS RELATIVE TO HUMAN IL-22**

(71) Applicant: Cytoki Pharma ApS, 2860 Søborg (DK)
(72) Inventor: Jørgensen, Rasmus, 2860 Søborg (DK); Norrild, Jens, 2860 Søborg (DK)
(74) Representative: EIP

(57) **Abstract**

Disclosed herein are tissue selective IL-22 agonists comprising specific amino acid modifications relative to human IL-22.

## Description

### FIELD OF THE INVENTION

The present invention relates to novel interleukin-22 (IL-22) protein variants of native mature human IL-22 (hereinafter "hlL-22"). The invention also encompasses methods for their production and their use in therapy, including the treatment, prevention, and/or amelioration of metabolic, pulmonary, gut, pancreatic, kidney, and/or central nervous system (CNS) diseases, disorders, and/or conditions.

### BACKGROUND OF THE INVENTION

IL-22 belongs to the IL-10 family of cytokines and is a 146-amino acid protein with a molecular weight of 17 KDa. Like other IL-10 family members, the IL-22 structure contains six α-helices (referred to as helices A to F). IL-22 is released in response to various stimuli and has been found to engage in tissue protection, repair acceleration, fibrosis prevention, and inflammation control.

IL-22 selectively activates a heterodimeric receptor (IL-22R) consisting of IL-22RA1 and IL-10RB subunits. The IL-10RB subunit is ubiquitously expressed, and the IL-22RA1 subunit has an epithelial-restricted expression. IL-22 is a unique cytokine in that it is released from immune cells but selectively targets epithelial cells. Hence, the signalling pathways induced by IL-22 may have relevance in different tissues (including skin, intestine, lung, liver, kidney, pancreas, colon, and thymus), but IL-22 activates them in an epithelial-specific manner. A soluble binding protein, IL-22BP, neutralises IL-22 and thus regulates its effect. hIL-22 is secreted primarily by CD4 T cells and group 3 innate lymphocytes (ILC3s).

In healthy individuals, low levels of IL-22 are secreted by immune cells, mainly in the gastrointestinal (GI) tract. However, much of this IL-22 is likely not biologically active due to the high levels of IL-22 binding protein (IL-22BP) produced by intestinal dendritic cells (DCs). IL-22BP is produced primarily by DCs, and is a homolog of IL-22R but lacks a transmembrane domain, allowing for secretion of IL-22BP. IL-22BP binds to IL-22 with up to 10,000 greater affinity than IL-22R and therefore in the presence of IL-22BP, most IL-22 binds to IL-22BP, inhibiting its ability to bind to the IL-22 receptor complex and cause changes in epithelial cells.

IL-22 is a critical cytokine in the modulation of tissue responses during inflammation and is highly upregulated in many chronic inflammatory disease patients, including those with psoriasis, rheumatoid arthritis, and inflammatory bowel disease (IBD). IL-22 has demonstrated protective effects and promotion of wound healing and tissue regeneration, while also being implicated in inducing adverse effects in the skin as an on-target effect.

Given that IL-22 induces signalling pathways in several different tissue targets, tissue specific action is typically limited. Consequentially, both desired and undesired tissue responses may occur and lead to unwanted side effects. A need therefore remains in the art for new variants of hIL-22, and other reference IL-22 proteins, which can induce signalling pathways in specific tissue over others thus activating beneficial IL-22R mediated effects while mitigating unwanted effects. This would improve the therapeutic properties of IL-22 and would enhance IL-22 proteins' potential as safe and effective pharmaceutical treatments for metabolic, pulmonary, gut, pancreatic, kidney, or central nervous system (CNS) diseases, disorders and/or conditions.

By addressing the need for improved IL-22 variants the inventors have developed potent modified IL-22 proteins, targeting the colon specifically with only low or no pharmaceutically relevant activity in other tissues like the skin and/or liver. These new, modified IL-22 proteins provide new and safe opportunities for targeted therapeutic interventions in multiple health conditions where IL-22 activity is desired in a more tissue specific application and thus provide a significantly enhanced outlook with better tolerability for patients in the need of treatment.

### SUMMARY OF THE INVENTION

A first aspect of the invention provides an IL-22 variant having a sequence identity of at least 90% to SEQ ID NO:1, comprising one or more amino acid substitution relative to SEQ ID NO: 1 in at least one of positions 19, 87 or 94. An example of the first aspect of the invention provides an IL-22 variant having a sequence identity of at least 90% to SEQ ID NO:1, comprising two or more amino acid substitution relative to SEQ ID NO: 1, including at least one in any one of positions 19, 87 or 94. An example of this first aspect, thus comprises at least two substitutions in any position selected from position 19, 87 or 94. One example of the first aspect of the invention provides an IL-22 variant having a sequence identity of at least 94% to SEQ ID NO:1, comprising an amino acid substitution relative to SEQ ID NO:1 in at least one of positions 19, 87 or 94. One example of the first aspect of the invention provides an IL-22 variant having a sequence identity of at least 95% to SEQ ID NO:1, comprising an amino acid substitution relative to SEQ ID NO: 1 in at least one of positions 19, 87 or 94. One example of the first aspect of the invention provides an IL-22 variant having a sequence identity of at least 96% to SEQ ID NO:1, comprising an amino acid substitution relative to SEQ ID NO: 1 in at least one of positions 19, 87 or 94. One example of the first aspect of the invention provides an IL-22 variant having a sequence identity of at least 97% to SEQ ID NO:1, comprising an amino acid substitution relative to SEQ ID NO: 1 in at least one of positions 19, 87 or 94. One example of the first aspect of the invention provides an IL-22 variant having a sequence identity of at least 98% to SEQ ID NO:1, comprising an amino acid substitution relative to SEQ ID NO: 1 in at least one of positions 19, 87 or 94. One example of the first aspect of the invention provides an IL-22 variant having a sequence identity of at least 99% to SEQ ID NO:1, comprising an amino acid substitution relative to SEQ ID NO: 1 in at least one of positions 19, 87 or 94.

In other words, the first aspect of the invention provides an IL-22 variant having at least one amino acid modifications and up to 15 or up to 8 amino acid modifications relative to SEQ ID NO: 1.

In some examples of the first aspect this means, that the invention provides an IL-22 variant having at least one amino acid modifications and up to 8 amino acid modifications, such as 7, 6, 5, 4, 3, 2 or 1 amino acid modifications relative to SEQ ID NO: 1. In some examples of the first aspect this means, that the invention provides an IL-22 variant having at least one amino acid modifications and up to 7 amino acid modifications, such as 6, 5, 4, 3, 2 or 1 amino acid modifications relative to SEQ ID NO: 1. In some examples of the first aspect this means, that the invention provides an IL-22 variant having at least one amino acid modifications and up to 6 amino acid modifications, such as 5, 4, 3, 2 or 1 amino acid modifications relative to SEQ ID NO: 1. In some examples of the first aspect this means, that the invention provides an IL-22 variant having at least one amino acid modifications and up to 5 amino acid modifications, such as 4, 3, 2 or 1 amino acid modifications relative to SEQ ID NO: 1. In some examples of the first aspect this means, that the invention provides an IL-22 variant having at least one amino acid modifications and up to 4 amino acid modifications, such as 3, 2 or 1 amino acid modifications relative to SEQ ID NO: 1. In some examples of the first aspect this means, that the invention provides an IL-22 variant having at least one amino acid modifications and up to 3 amino acid modifications, such as 2 or 1 amino acid modifications relative to SEQ ID NO: 1. In some examples of the first aspect this means, that the invention provides an IL-22 variant having at least one amino acid modifications and up to 6 amino acid modifications, such as 5, 4, 3, 2 or 1 amino acid modifications relative to SEQ ID NO: 1.

In some examples of the first aspect this means, that the invention provides an IL-22 variant having at least two amino acid modifications and up to 8 amino acid modifications, such as 7, 6, 5, 4, 3 or 2 amino acid modifications relative to SEQ ID NO: 1. In some examples of the first aspect this means, that the invention provides an IL-22 variant having at least two amino acid modifications and up to 7 amino acid modifications, such as 6, 5, 4, 3 or 2 amino acid modifications relative to SEQ ID NO: 1. In some examples of the first aspect this means, that the invention provides an IL-22 variant having at least two amino acid modifications and up to 6 amino acid modifications, such as 5, 4, 3 or 2 amino acid modifications relative to SEQ ID NO: 1. In some examples of the first aspect this means, that the invention provides an IL-22 variant having at least two amino acid modifications and up to 5 amino acid modifications, such as 4, 3 or 2 amino acid modifications relative to SEQ ID NO: 1. In some examples of the first aspect this means, that the invention provides an IL-22 variant having at least two amino acid modifications and up to 4 amino acid modifications, such as 3 or 2 amino acid modifications relative to SEQ ID NO: 1. In some examples of the first aspect this means, that the invention provides an IL-22 variant having at least two amino acid modifications and up to 3 amino acid modifications.

In some examples of the first aspect this means, that the invention provides an IL-22 variant having at least three amino acid modifications and up to 8 amino acid modifications, such as 7, 6, 5, 4 or 3 amino acid modifications relative to SEQ ID NO: 1. In some examples of the first aspect this means, that the invention provides an IL-22 variant having at least three amino acid modifications and up to 7 amino acid modifications, such as 6, 5, 4 or 3 amino acid modifications relative to SEQ ID NO: 1. In some examples of the first aspect this means, that the invention provides an IL-22 variant having at least three amino acid modifications and up to 6 amino acid modifications, such as 5, 4 or 3 amino acid modifications relative to SEQ ID NO: 1. In some examples of the first aspect this means, that the invention provides an IL-22 variant having at least three amino acid modifications and up to 5 amino acid modifications, such as 4 or 3 amino acid modifications relative to SEQ ID NO: 1. In some examples of the first aspect this means, that the invention provides an IL-22 variant having at least three amino acid modifications and up to 4 amino acid modifications.

In some cases, the invention provides an IL-22 variant, wherein
a. the amino acid Isoleucine (I) of position 19 of SEQ ID NO:1 is substituted with:
   i. Alanine (A), Valine (V), or Leucine (L)
   ii. a non-native hydrophobic amino acid; or
   iii. suitably a hydrophobic amino acid comprising an aromatic ring;
   and/or
b. the amino acid Proline (P) of position 87 of SEQ ID NO:1 is substituted with
   i. Alanine (A), Valine (V), Isoleucine (I) or Leucine (L);
   ii. a hydrophobic amino acid;
   iii. suitably a hydrophobic amino acid comprising an aromatic ring; or
   iv. a positively charged amino acid;
   and/or
c. the amino acid Asparagine (N) of position 94 of SEQ ID NO:1 is substituted with
   i. Alanine (A), Valine (V), Isoleucine (I) or Leucine (L);
   ii. a hydrophobic amino acid; or
   iii. suitably a hydrophobic amino acid comprising an aromatic ring.

In some cases, the invention provides an IL-22 variant, wherein
a. the amino acid Isoleucine (I) of position 19 of SEQ ID NO:1 is substituted with
   i. a non-native hydrophobic amino acid; or
   ii. suitably a hydrophobic amino acid comprising an aromatic ring;
   and/or
b. the amino acid Proline (P) of position 87 of SEQ ID NO:1 is substituted with
   i. a hydrophobic amino acid;
   ii. suitably a hydrophobic amino acid comprising an aromatic ring; or
   iii. a positively charged amino acid;
   and/or
c. the amino acid Asparagine (N) of position 94 of SEQ ID NO:1 is substituted with
   i. a hydrophobic amino acid; or
   ii. suitably a hydrophobic amino acid comprising an aromatic ring.

In some cases, the invention provides an IL-22 variant, wherein
a. the amino acid Isoleucine (I) of position 19 of SEQ ID NO:1 is substituted with a Alanine (A), Valine (V), Leucine (L), Phenylalanine (F) or Tryptophan (W); and/or
b. the amino acid Proline (P) of position 87 of SEQ ID NO:1 is substituted with Valine (V), Isoleucine (I), Leucine (L), Arginine (R), Histidine (H), Lysine (K), Tryptophane (W) or Phenylalanine (F); and/or
c. the amino acid Asparagine (N) of position 94 of SEQ ID NO:1 is substituted with Alanine (A), Valine(V), Isoleucine (I), Leucine (L), Phenylalanine (F), or Tryptophan (W), Histidine (H).

In some cases, the invention provides an IL-22 variant, wherein
a. the amino acid Isoleucine (I) of position 19 of SEQ ID NO:1 is substituted with a Phenylalanine (F) or Tryptophan (W); and/or
b. the amino acid Proline (P) of position 87 of SEQ ID NO:1 is substituted with Valine (V), Isoleucine (I), Leucine (L), Arginine (R), Histidine (H), Tryptophane (W) or Phenylalanine (F); and/or
a. the amino acid Asparagine (N) of position 94 of SEQ ID NO:1 is substituted with Phenylalanine (F) or Tryptophan (W).

In some cases, the invention provides an IL-22 variant, wherein
a. the amino acid Isoleucine (I) of position 19 of SEQ ID NO:1 is substituted with Phenylalanine (F); and/or
b. the amino acid Proline (P) of position 87 of SEQ ID NO:1 is substituted with Arginine (R), Phenylalanine (F), and/or
c. the amino acid Asparagine (N) of position 94 of SEQ ID NO:1 is substituted with Phenylalanine (F).

In some cases, the invention provides an IL-22 variant, comprising at least one amino acid substitution selected from the group consisting of I19F, P87R or N94F. In some cases, the invention provides an IL-22 variant, comprising at least one amino acid substitution selected from the group consisting of I19F, P87F or N94F.

In some cases, the IL-22 variant of the invention further comprises one or more amino acid deletion, insertions or extensions, and/or one or more further amino acid substitutions, all relative to SEQ ID NO:1.

In some cases, the IL-22 variant of the invention further comprises at least one amino acid substitution selected from S12E, N13A, Q83A, E84A, R91A or R95A.

In some cases, the IL-22 variant of the invention further comprises at least one amino acid substitution selected from A1G, A1H, N35D, N35H, N35Q, N64D, N64Q, N64W, A90F, Q113R, and K114R, suitably selected from N35Q and N64Q.

In some cases, the IL-22 variant of the invention is a variant of native mature human IL-22 (hIL-22; SEQ ID NO. 1) and the variant comprises 1-10, 1-8 or 1-6 amino acid substitutions. In some such cases, the variant additionally comprises an N-terminal peptide and/or a C-terminal peptide extension.

A second aspect of the invention provides a derivative of IL-22 comprising an IL-22 variant according to the first aspect, and wherein the derivatization comprises one or more of (i) a covalently attached fatty acid, (ii) a covalently attached PEG group and (iii) formation of an Fc-fusion protein comprising the IL-22 variant and at least a portion of an antibody Fc protein.

A third aspect of the invention provides a pharmaceutical composition comprising an IL-22 variant according to the first aspect or a derivative according to the second aspect, and a pharmaceutically acceptable vehicle, wherein the pharmaceutical composition is suitable for administration by inhalation, administration by injection, for oral administration, rectal administration or ocular administration, optionally wherein the administration by injection is intraperitoneal, intramuscular, subcutaneous or intravenous.

The invention also provides an IL-22 variant, a derivative or a pharmaceutical composition according to the first, second or third aspect of the invention, for use in therapy.

The invention also provides an IL-22 variant, a derivative or a pharmaceutical composition according to the first, second or third aspect of the invention, for use in a method of treating a metabolic, pulmonary, gut, pancreatic, kidney, or central nervous system (CNS) disease, disorder and/or condition.

In some cases, the variant, derivative or pharmaceutical composition is for use in treating a disease, disorder and/or condition, wherein:
(i) the metabolic disease, disorder and/or condition is obesity, prediabetes, insulin resistance, diabetes type 1, diabetes type 2, metabolic dysfunction-associated steatohepatitis (MASH), hyperlipidaemia, hyperglycaemia or hyperinsulinemia;
(ii) the pulmonary disease, disorder and/or condition is chronic obstructive pulmonary disease (COPD), cystic fibrosis, bronchiectasis, idiopathic pulmonary fibrosis, acute respiratory distress syndrome, a chemical injury, a viral infection, a bacterial infection or a fungal infection;
(iii) the gut disease, disorder and/or condition is inflammatory bowel disease (IBD), ulcerative colitis, Crohn's disease, graft-versus-host-disease (GvHD), coeliac disease, a chemical injury, a viral infection or a bacterial infection;
(iv) the pancreatic disease, disorder and/or condition is pancreatitis;
(v) the kidney disease, disorder and/or condition is acute kidney disease or chronic kidney disease; or
(vi) the CNS disease, disorder and/or condition is multiple sclerosis.

### BRIEF DESCRIPTION OF THE DRAWINGS

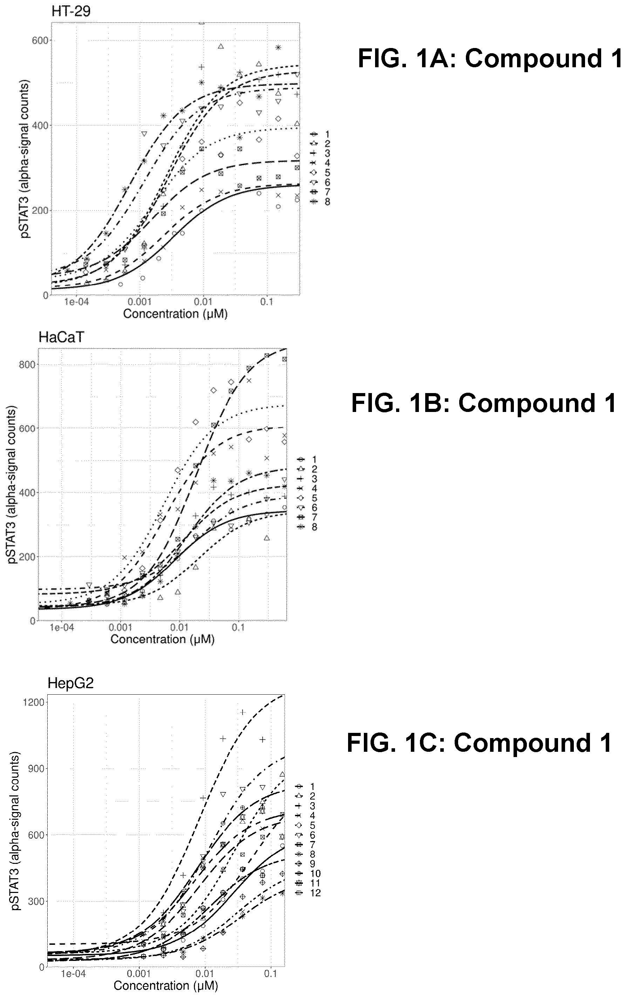

**Figure 1****:** illustrates the dose-response curves for STAT3 phosphorylation after stimulation with compound 1 (wild-type human IL-22 / SEQ ID NO:1) in three different cell lines. Figure 1A shows the dose-response curve for the tested compound's effect on STAT3 phosphorylation in the HT-29 cells (colon cell line) and includes data from 8 experiments, Figure 1B shows the dose-response curve for the tested compound's effect on STAT3 phosphorylation in the HaCaT cells (skin cell line) and includes data from 8 experiments, Figure 1C shows the dose-response curve for the tested compound's effect on STAT3 phosphorylation in the HepG2 cells (liver cell line) and includes data from 12 experiments. Compound 1 (or wild-type human IL-22) shows greater potency in the colon cell line versus the skin and liver cell lines.

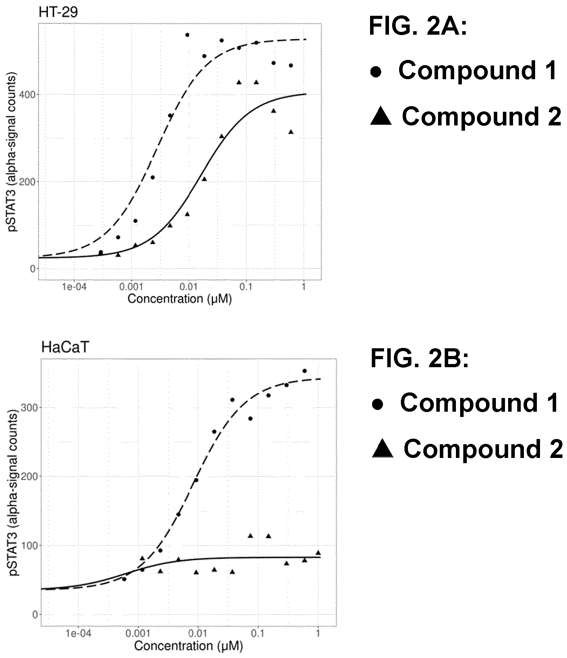

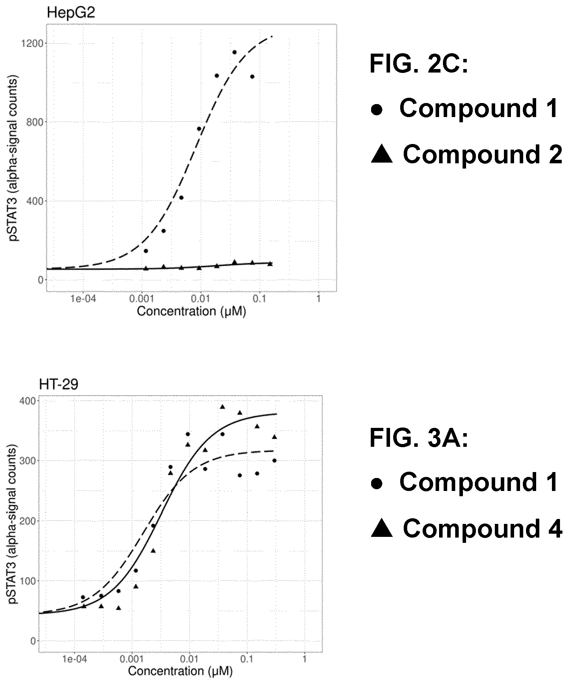

**Figure 2****:** illustrates the dose-response curves for STAT3 phosphorylation after stimulation with compound 1 (wild-type human IL-22 / SEQ ID NO:1) (•) and compound 2 (▲) in three different cell lines. Figure 2A shows the dose-response curve for the tested compounds' effect on STAT3 phosphorylation in the HT-29 cells (colon cell line), Figure 2B shows the dose-response curve for the tested compounds' effect on STAT3 phosphorylation in the HaCaT cells (skin cell line), Figure 2C shows the dose-response curve for the tested compounds' effect on STAT3 phosphorylation in the HepG2 cells (liver cell line). This series of figures illustrates the pronounced effect of the P87R amino acid substitution, which eliminates (or reduces) the activity of the IL-22 variant in the liver and skin cell lines but remains active in the colon cell lines.

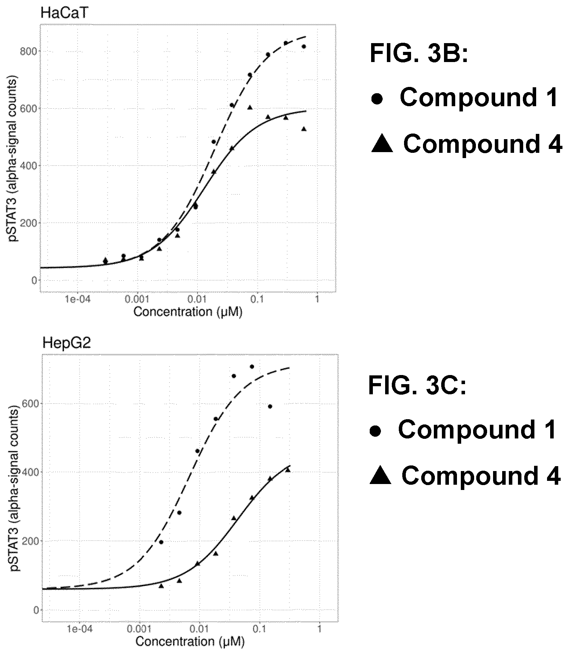

**Figure 3****:** illustrates the dose-response curves for STAT3 phosphorylation after stimulation with compound 1 (wild-type human IL-22 / SEQ ID NO:1) (•) and compound 4 (▲) in three different cell lines. Figure 3A shows the dose-response curve for the tested compounds' effect on STAT3 phosphorylation in the HT-29 cells (colon cell line), Figure 3B shows the dose-response curve for the tested compounds' effect on STAT3 phosphorylation in the HaCaT cells (skin cell line), Figure 3C shows the dose-response curve for the tested compounds' effect on STAT3 phosphorylation in the HepG2 cells (liver cell line). Compound 4 is an IL-22 variant comprising the amino acid modification P87F which is potent and efficacious in the colon cell line yet leads to reduced efficacy and/or potency in liver and skin cell lines.

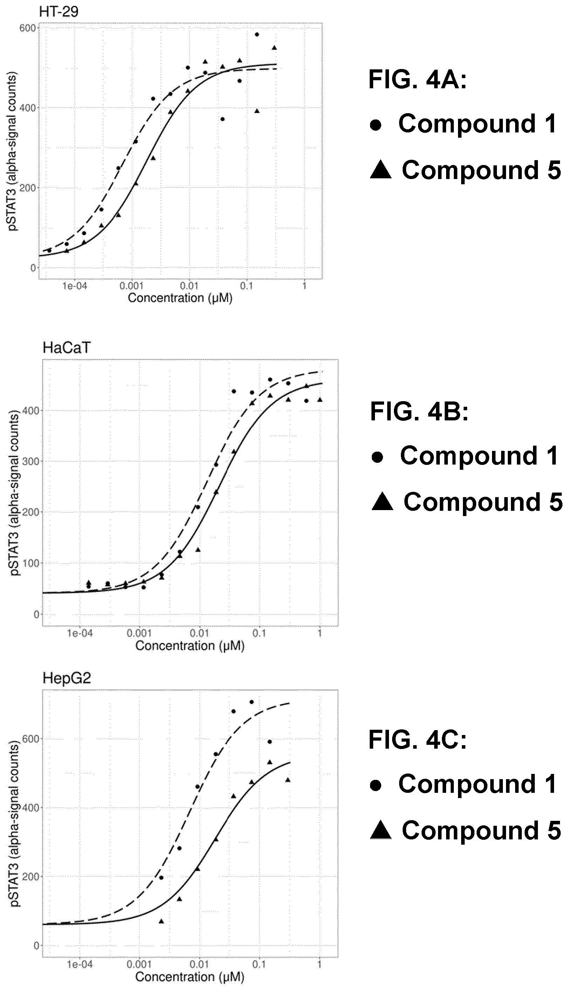

**Figure 4****:** illustrates the dose-response curves for STAT3 phosphorylation after stimulation with compound 1 (wild-type human IL-22 / SEQ ID NO:1) (•) and compound 5 (▲) in three different cell lines. Figure 4A shows the dose-response curve for the tested compounds' effect on STAT3 phosphorylation in the HT-29 cells (colon cell line), Figure 4B shows the dose-response curve for the tested compounds' effect on STAT3 phosphorylation in the HaCaT cells (skin cell line), Figure 4C shows the dose-response curve for the tested compounds' effect on STAT3 phosphorylation in the HepG2 cells (liver cell line). Compound 5 is an IL-22 variant comprising the amino acid modification A90F which shows no bias towards any of the three tissues, i.e. is potent and efficacious in the colon, liver and skin cell lines.

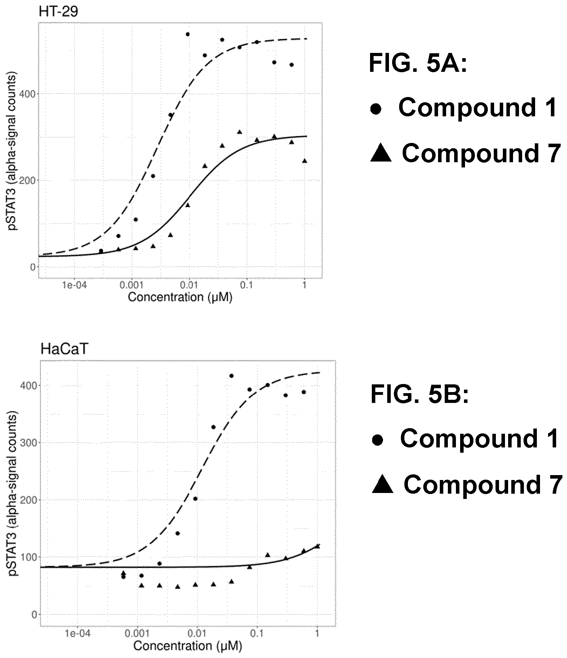

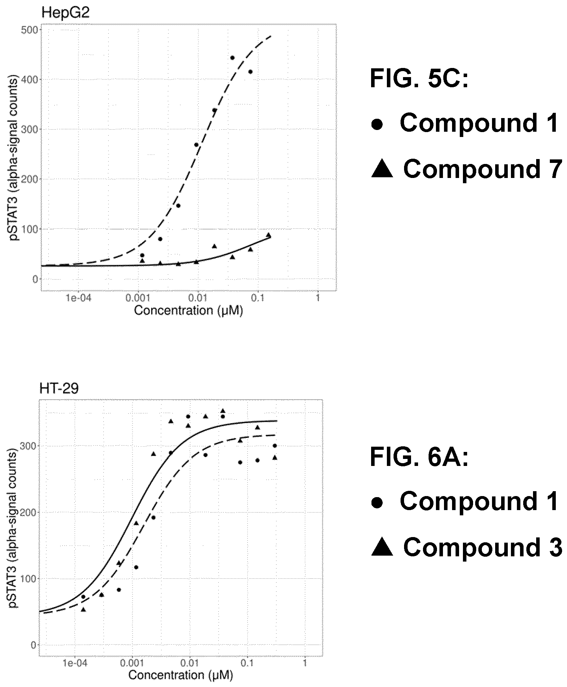

**Figure 5****:** illustrates the dose-response curves for STAT3 phosphorylation after stimulation with compound 1 (wild-type human IL-22 / SEQ ID NO:1) (•) and compound 7 (▲) in three different cell lines. Figure 5A shows the dose-response curve for the tested compounds' effect on STAT3 phosphorylation in the HT-29 cells (colon cell line), Figure 5B shows the dose-response curve for the tested compounds' effect on STAT3 phosphorylation in the HaCaT cells (skin cell line), Figure 5C shows the dose-response curve for the tested compounds' effect on STAT3 phosphorylation in the HepG2 cells (liver cell line). Compound 7 is an IL-22 variant comprising the amino acid modifications P87R and A90F, showing that the pronounced effect of the amino acid modification P87R on the elimination (or reduction) of the IL-22 variant effect in liver and skin is maintained, even when it is combined with a relatively non-tissue-specific amino acid modification A90F.

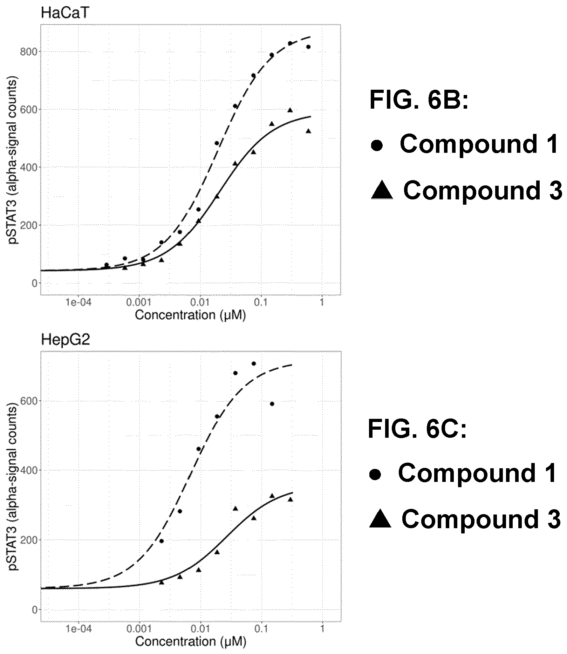

**Figure 6****:** illustrates the dose-response curves for STAT3 phosphorylation after stimulation with compound 1 (wild-type human IL-22 / SEQ ID NO:1) (•) and compound 3 (▲) in three different cell lines. Figure 6A shows the dose-response curve for the tested compounds' effect on STAT3 phosphorylation in the HT-29 cells (colon cell line), Figure 6B shows the dose-response curve for the tested compounds' effect on STAT3 phosphorylation in the HaCaT cells (skin cell line), Figure 6C shows the dose-response curve for the tested compounds' effect on STAT3 phosphorylation in the HepG2 cells (liver cell line). Compound 3 comprises the amino acid modification I19F and leads to an IL-22 variant which is potent and efficacious in the colon cell line, yet less potent in the skin and liver cell lines.

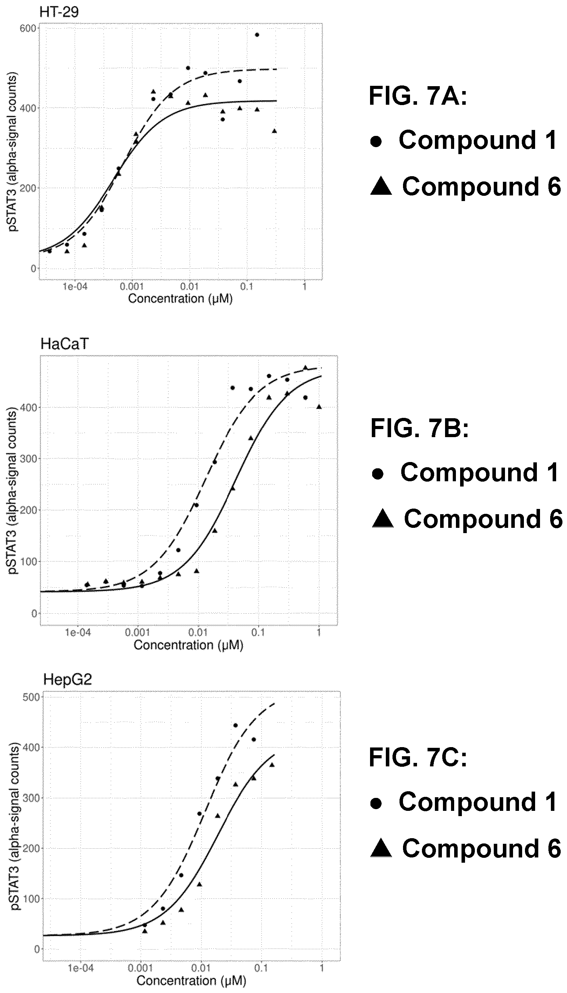

**Figure 7****:** illustrates the dose-response curves for STAT3 phosphorylation after stimulation with compound 1 (wild-type human IL-22 / SEQ ID NO:1) (•) and compound 6 (▲) in three different cell lines. Figure 7A shows the dose-response curve for the tested compounds' effect on STAT3 phosphorylation in the HT-29 cells (colon cell line), Figure 7B shows the dose-response curve for the tested compounds' effect on STAT3 phosphorylation in the HaCaT cells (skin cell line), Figure 7C shows the dose-response curve for the tested compounds' effect on STAT3 phosphorylation in the HepG2 cells (liver cell line). Compound 6 is an IL-22 variant comprising the amino acid modification N94F and elicits a substantially higher potency in the colon cell line over both the skin and liver cell lines.

### DETAILED DESCRIPTION

As will be discussed, the present invention relates to variants of reference IL-22 proteins, such as human IL-22 (hIL-22 - also referred to herein as wild type (wt) IL-22), which provide tissue specific action by significantly reducing signalling in the skin and liver whilst maintaining a good level of signalling in the gastrointestinal tract (all relative to signalling strength of hIL-22). The variants retain the intestinal functions of IL-22 in vitro with reduced or with no activity in the skin and liver thereby increasing the therapeutic window for targeted treatment in the desired tissue.

Accordingly, the amino acid substitutions in the hIL-22 polypeptide variants disclosed herein result in a tissue-selective IL-22 signalling which involves a reduction of IL-22 signalling in the skin and/or liver (relative to hIL-22) alongside substantial retention (or a proportionally smaller reduction) of IL-22 signalling in the gastrointestinal tract (relative to hIL-22).

The variants can also be derivatised. Also disclosed is a pharmaceutical composition comprising the variant (or derivative thereof). Also disclosed is the variant, derivative or pharmaceutical composition for use in a method of therapy, such as for treating a metabolic, pulmonary, gut, pancreatic, kidney and/or CNS disease, disorder, and/or condition.

### DEFINITIONS

In what follows, Greek letters are represented by their symbol rather than their written name. For example, α = alpha, ε = epsilon, γ = gamma and µ = mu. Amino acid residues may be identified by their full name, three-letter code or one-letter code, all of which are fully equivalent.

The term, **"IL-22 protein",** as used herein, can mean a native IL-22 protein, such as hIL-22 or murine IL-22, or a variant thereof.

The term **"variant of IL-22",** as used herein, refers to an IL-22 protein in which one or more amino acid substitutions, deletions, and insertions, or combinations thereof, are present as compared to the native IL-22 protein, such as native mature human IL-22 (hIL-22), SEQ ID NO:1.

The term **"amino acid modification",** as used herein includes any modification of the native IL-22 protein amino acid sequence, such as a substitution, deletion or insertion as defined below.

The term **"substitution",** as used herein, can mean the replacement of an amino acid in the native IL-22 protein with another. A substitution may be a conservative or a non-conservative substitution. The substitution herein may comprise the replacement of an amino acid from the native IL-22 sequence with a naturally occurring amino acid (a "natural amino acid") or a synthetic amino acid (a "non-natural amino acid"). In some preferred cases, the substitution comprises the replacement of an amino acid from the native IL-22 sequence with a natural amino acid. Substitutions are defined herein relative to the native IL-22; in other words, the site of substitution in the native protein is identified, and the replacement amino acid is indicated also. In some cases, an amino acid at a position in the native IL-22 is substituted with one or more amino acids. In some cases, said substitution with one or more amino acids is thus understood as a replacement of one amino acid relative to native IL-22 (h IL-22, SEQ ID NO:1) with another single amino acid. In some cases, said substitution with one or more amino acids is thus understood as a replacement of one amino acid relative to native IL-22 (h IL-22, SEQ ID NO:1) with more than one amino acid, such as a repetitive sequence of a single amino acids. In cases where one amino acid relative to native IL-22 is substituted with more than one amino acids, this substitution should be counted as one substitution. In cases were one amino acid relative to native IL-22 is substituted with a repetitive sequence of a single amino acids, this substitution should be counted as one substitution.

The term **"deletion",** as used herein, can mean the removal of an amino acid relative to the native IL-22 protein. The term **"insertion",** as used herein, can mean the addition of an amino acid relative to the native IL-22 protein. The term **"extension",** as used herein, can mean the addition of one or more amino acids to the N- or C-terminal of a native IL-22 protein, or an IL-22 variant as disclosed herein.

Expressions such as **"relative to",** are used herein to characterise the site of change inside or outside an IL-22 variant by reference to the sequence identity of the native protein, e.g. hIL-22. For example, a Glu substitution for the native Asp at residue 10 in hIL-22 is represented herein as "D10E". Expressions such as "within" are used herein to characterise the site of change inside an IL-22 protein sequence by reference to the sequence identity of the native IL-22 protein, e.g. hIL-22. The amino acid sequence of hIL-22 is identified herein as SEQ ID NO. 1, where the first amino acid residue of hIL-22 (Alanine (Ala)) is assigned position 1. Thus, a variation "within" the sequence of hIL-22 is a variation to any of residue numbers 1 -146 in SEQ ID NO. 1.

Other variations outside of the native protein sequence are contemplated, e.g. a variation external to residue numbers 1-146 in hIL-22 SEQ ID NO. 1. For example, a variant as defined herein may include an N-terminal peptide, which may, for example, be of 3 amino acids in length. To identify sites within such N-terminal extensions relative to the native protein, the residues in the N-terminal peptide are numbered negatively, starting from the residue attached to residue 1 in e.g. hIL-22, i.e. the first residue in the N-terminal peptide that is attached to residue 1 in hIL-22 is denoted "-1". Naturally, however, the numbering used in the sequence listing for such a variant would start from 1, in accordance with WIPO Standard ST.26; as such, for the example position 1 in the sequence listing for a variant with a trimer N-terminal peptide would be residue -3 as referred to herein. Amino acids in C-terminal peptide extensions are numbered sequentially relative to the native protein and continuing from the native IL-22; so, for a C-terminal extension to hIL-22, the first amino acid attached to the end of the IL-22 is numbered 147.

The term **"derivative of IL-22",** as used herein, refers to an hIL-22 variant having one or more of (i) a covalently attached fatty acid, and (ii) a covalently attached PEG group; or alternatively refers to an Fc-fusion protein, which is a recombinant protein comprising the IL-22 protein and at least a portion of an antibody Fc protein. The fatty acid or PEG group may, in some instances, be covalently attached at the amino acid insertion or substitution position on the IL-22 variant or attached to the N-terminal or C-terminal peptide. The term derivative encompasses both derivatives in which the fatty acid or PEG group is covalently attached to the IL-22 protein directly and those in which the covalent attachment is by a linker.

The term **"EC₅₀",** as used herein, refers to the concentration of drug required to produce 50% of the maximal effect. The half maximal effective concentration (EC₅₀) value is often used as a measure of the potency of a drug, wherein the lower the EC₅₀ value of a drug, the higher the potency. Potency is the amount of drug needed to produce a certain response. It depends on the affinity of the drug to the binding site, as well as the efficacy it can elicit. The less potent a drug is, the higher the dose needed to reach its effect. The EC₅₀ for any drug is tissue-specific, as different tissues have different sensitivities to the drug (in part due to tissue specific receptor expression). Furthermore, EC₅₀ is dependent on many factors including species, tissue and cell type and genetics. The use of human cell lines to test a drug candidate's in vitro potency (EC₅₀) is one of the early and decisive components to choose the dose of a drug candidate to develop further. A preference for activity in one tissue/cell type over another can present as a lower EC₅₀ in a preferred cell line relative to another cell line.

The term **"Eₘₐₓ",** as used herein, refers to the maximum effect of a drug. Efficacy is the ability of a drug to elicit a response when it interacts with a receptor. It depends on the concentration at the site of action, the number of drug-receptor binding sites, psychological factors, and the efficiency of the coupling of receptor activation to cellular responses. The Eₘₐₓ for any drug is tissue-specific, as different tissues having different sensitivities to the drug (in part due to tissue specific receptor expression). Furthermore, Eₘₐₓ is dependent on many factors including species, tissue and cell type and genetics. The use of human cell lines to test a drug candidate's in vitro efficacy (expressed as Eₘₐₓ) is one of the early and decisive components to choose the dose of a drug candidate to develop further.

A **"wild-type relative Eₘₐₓ**" (% activity of hIL-22) is calculated by dividing the Eₘₐₓ of the variant by the Eₘₐₓ of the wild-type hIL-22 (control) used in the experiment after subtraction of the background signal. When a compound has a reduced Eₘₐₓ compared to the wild-type hIL-22 (full agonist) it is referred to as a partial agonist and has a wild-type relative Eₘₐₓ value of less than 100%.

The term **"tissue selectivity"** or **"tissue specificity"** or the like, as used herein in the context of a variant, means that the response induced by that variant is biased, inducing a greater response in one tissue type over another i.e. a preference for one tissue/cell line over another. Typically, the bias is greater than the bias for wild-type hIL-22. Tissue selectivity may arise from (a) different efficacies (Eₘₐₓ values) in the respective tissues, or (b) different potencies (EC₅₀ values) in the respective tissues, such that at a given drug concentration, a greater response is induced in one tissue over another, or (c) a combination of (a) and (b).

Note that establishing the actual Eₘₐₓ requires the Eₘₐₓ to be reached in the tested concentration range. For compounds with a high EC₅₀ (low potency agonists) in a particular cell line, the actual Eₘₐₓ may fall outside the tested concentration range, potentially falsely presenting as partial agonists. However, testing higher concentrations in in vitro cell assays may not be feasible because of technical constraints (analogue solubility, etc.) and, importantly, would be outside meaningful pharmacological concentrations for drug development. Regardless, conclusions may be drawn about tissue selectivity, irrespective of whether the selectivity presents as relative change in potency ratio or as partial agonism, as discussed in more detail below.

The terms **"naturally occurring amino acid"** or **"natural amino acid"** refer to the group of amino acids consisting of: Arginine (R), Histidine, Lysine(K), Aspartic Acid (D), Glutamic Acid (E), Serine (S), Threonine (T), Asparagine (N), Glutamine (Q), Cysteine (C), Selenocysteine (U), Glycine (G), Proline (P), Alanine (A), Valine(V), Isoleucine (I),Leucine (L), Methionine (M), Phenylalanine (F), Tyrosine (Y), Tryptophan (W). These are typically classified into (i) charged amino acids, (ii) polar, uncharged amino acids, (iii) hydrophobic amino acids, and (iv) special case amino acids. Specifically, Arginine (R), Histidine (H), Lysine (K), Aspartic Acid (D), and Glutamic acid (E) are known as "charged amino acids", with Arginine (R), Histidine (H) and Lysine (K) being positively charged. Serine (S), Threonine (T), Asparagine (N) and Glutamine (Q) are known as "polar, uncharged amino acids"; of these, Asparagine (A) and Glutamine (Q) are considered "large, polar, uncharged amino acids", each having 5 carbons in the backbone. Cysteine (C), Selenocysteine (U), Glycine (G) and Proline (P) are known as "special case amino acids". Alanine (A), Valine(V), Isoleucine (I),Leucine (L), Valine (V), Isoleucine (I), Leucine (L), Methionine (M), Phenylalanine (F), Tyrosine (T) and Tryptophan (W) are known as "hydrophobic amino acids"; of these, Methionine (M), Phenylalanine (F), Tyrosine (T) and Tryptophan (W) are considered "large hydrophobic amino acids", having sulphide or aromatic groups in the side chain.

Conservative amino acid substitutions are the exchange of an amino acid for another in the same class (e.g. substituting a hydrophobic amino acid for another hydrophobic amino acid). Non-conservative substitutions are the exchange of an amino acid for another in a different class (e.g. substituting a hydrophobic amino acid for a charged amino acid).

The terms **"non-naturally occurring amino acid", "synthetic amino acid"** or **"non-natural amino acid"** as used herein, referto amino acids suitable for use in pharmaceutical peptides or proteins, which are not selected from the group of naturally occurring amino acids, consisting of: Arginine, Histidine, Lysine, Aspartic Acid, Glutamic Acid, Serine, Threonine, Asparagine, Glutamine, Cysteine, Selenocysteine Glycine, Proline, Alanine, Valine, Isoleucine, Leucine, Methionine, Phenylalanine, Tyrosine, Tryptophan. "Non-natural amino acids" are often used in the development of pharmaceutical peptides or proteins and may be used in place of, or in addition to, the naturally occurring amino acids already present in the amino acid sequence of a given peptide or protein, which is improved for a specific pharmaceutical application. Such "non-natural amino acids" are often used to achieve specific function in addition to their function as amino acid per se, e.g., to utilize non-proteinogenic or other stabilizing properties. "Non-natural amino acids" are commonly known to include, but not to be limited to non-natural amino acids selected from the group consisting of: D-amino acids, β-amino acids, N-methyl amino acids, α,α-disubstituted amino acids, Unnatural aromatic amino acids (e.g., D-phenylalanine), Norleucine, Hydroxyproline, Phosphoserine, 4-fluorophenylalanine, Azidohomoalanine. Specific examples of often used "non-natural amino acids" in the development of pharmaceutical peptides or protein drug candidates are selected from the group consisting of: Aib (a-aminoisobutyric acid), Boc-lysine (t-butoxycarbonyl), Fmoc-lysine (9-fluorenylmethyloxycarbonyl), Homoserine, 4-aminophenylalanine and Beta-alanine.

A **"native"** amino acid, refers to the amino acid in wild-type h-IL22, i.e. the amino acids of SEQ ID NO. 1. A **"non-native"** amino acid refers to an amino acid at a given site in the peptide chain being different from the native or wild-type sequence. Thus, indication that an amino acid at any given site is "non-native" means that the amino acid at that site is different from, or modified relative to, SEQ ID NO.1.

A **"therapeutically effective amount"** of a variant of the invention is any amount which, when administered to a subject, is the amount of variant that is needed to treat the disease, disorder and/or condition or produce the desired effect.

A **"pharmaceutically acceptable vehicle"** as referred to herein, is any known compound or combination of known compounds that are known to those skilled in the art to be useful in formulating pharmaceutical compositions.

Terms such as **"treating"** and **"therapy",** as used herein, expressly include the treatment, amelioration and/or prevention of a disease, disorder, and/or condition.

A **"subject"** or **"patient"**, as used herein, may be a vertebrate, mammal, or domestic animal. Hence, variants and compositions according to the invention may be used to treat any mammal, for example livestock (for example, a horse), pets, or may be used in other veterinary applications. In some cases, the subject or patient may be human.

### IL-22 VARIANTS

The invention provides an IL-22 variant having a sequence identity of at least 90% to SEQ ID NO:1, comprising an amino acid substitution relative to SEQ ID NO:1 in at least one of positions 19, 87 or 94. In one case, the invention thus provides such an IL-22 variant with at least 93% to SEQ ID NO:1. In one case, the invention thus provides such an IL-22 variant with at least 94% to SEQ ID NO:1. In one case, the invention thus provides such an IL-22 variant with at least 95% to SEQ ID NO:1. In one case, the invention thus provides such an IL-22 variant with at least 96% to SEQ ID NO:1. n one case, the invention thus provides such an IL-22 variant in which at least one of positions 19, 87 or 94 is a non-native amino acid relative to SEQ ID NO: 1 In one case, the invention thus provides such an IL-22 variant with at least 98% to SEQ ID NO:1. In one case, the invention thus provides such an IL-22 variant with at least 99% to SEQ ID NO:1.

The substitutions are relative to SEQ ID NO:1, so the IL-22 variant has an amino acid at position 19 and/or 87 and/or 94 which does not match the amino acid in that position in SEQ ID NO:1.

In other words, the invention provides a variant of hIL-22 comprising an amino acid substitution in at least one of positions 19, 87 or 94, further optionally comprising amino acid modifications, such as substitutions in other positions than said positions 19, 87 or 94, provided that the sequence identity of at least 90%, at least 94%, at least 95%, at least 96%, at least 97%, at least 98%, at least 98% or at least 99% to SEQ ID NO:1. The variant may additionally include one or more amino acid sequence variations outside the native sequence (e.g. N- or C-terminal peptide extensions). Said variants can be derivatised.

In some cases, the variant has at least two modifications, such as substitutions, relative to SEQ ID NO:1.

The inventors have found that IL-22 variants with a substitution (relative to SEQ ID NO:1) in at least one of positions 19, 87 and 94 provide tissue selectivity. The inventors have found that IL-22 variants with a substitution (relative to SEQ ID NO:1) in two of positions 19, 87 and 94 provide tissue selectivity. Such variants activate signalling pathways in the colon more strongly than in the skin and/or liver. The bias is greater than that seen in hIL-22. Tissue selectivity is present where either or both:
- The IL-22 variant's efficacy in the colon is greater than in skin or liver; that is, the variant triggers a stronger signalling response in the colon. This can be characterised as Eₘₐₓ(colon) > Eₘₐₓ(skin) and Eₘₐₓ(colon) > Eₘₐₓ(liver).
- The IL-22 variant's potency in the colon is greater than in the skin or liver; that is, the variant triggers a signalling response in the colon at lower concentrations than required to trigger a signalling response in the skin or liver. This can be characterised as EC₅₀(colon) < EC₅₀(skin) and EC₅₀(colon) < EC₅₀(liver).

Without being bound by theory, it is believed that positions 19, 87 and 94 of hIL-22 interact with IL-10R2 subunit in the heterodimeric receptor and substitutions at positions 19, 87 and 94 weaken the interaction of the variant with IL-10R2 relative to wild-type hIL-22. IL-10R2 expression is stronger in the colon than in the skin and liver. Variant activity corresponds to IL-10R2 expression, and so the IL-22 variants with substitutions at these sites induce signalling in colon tissue with low or no pharmaceutically relevant signalling in skin and liver tissues.

Suitably, an IL-22 variant of the first aspect comprises 200 amino acids or less. For example, the variant comprises less than 190, less than 180, less than 170, less than 160 or even less than 150 amino acids. Suitably, the variant will comprise at least 146 amino acids, however, this being the number of amino acids in hIL-22. It may comprise at least 150 amino acids, at least 160 amino acids, at least 170 amino acids or even at least 180 amino acids. The variant of the invention can comprise variants of any length within the above ranges, but they will typically be 146-180 amino acids in length.

In some instances, an IL-22 variant of the first aspect has a sequence identity of at least 90%, 92%, 93%, 94%, 95%, 96%, 97%, 98% or 99% to SEQ ID NO:1. In some instances, an IL-22 variant of the first aspect has a sequence identity of between 97% and 99.5% to SEQ ID NO:1.

In some cases, the protein is a variant of native mature human IL-22 (hIL-22; SEQ ID NO. 1) and the variant comprises 1-10 amino acid modifications, such as substitutions, such as 1, 2, 3, 4, 5, 6, 7, 8, 9 or 10 amino acid modifications or substitutions, preferable between 1 and 8 or 1 and 6.

In some cases, the protein is a variant of native mature human IL-22 (hIL-22; SEQ ID NO. 1) and the variant comprises 1 to 8 amino acid substitutions relative to SEQ ID NO:1. In some cases, the protein is a variant of native mature human IL-22 (hIL-22; SEQ ID NO. 1) and the variant comprises 1 to 6 amino acid substitutions relative to SEQ ID NO:1. In some cases, the protein is a variant of native mature human IL-22 (hIL-22; SEQ ID NO. 1) and the variant comprises 1 to 3 amino acid modifications, such as substitutions relative to SEQ ID NO:1. In some examples, such variants comprising 1 to 8 amino acid modifications, such as substitutions relative to SEQ ID NO: 1, may optionally and additionally comprise amino acid extensions in the C-terminal or N-terminal. In some examples, such variants comprising 1 or more amino acid modifications, such as substitutions relative to SEQ ID NO: 1, may optionally and additionally comprise amino acid extensions in the C-terminal or N-terminal. In some examples, such variants comprising 2 or more amino acid modifications, such as substitutions relative to SEQ ID NO: 1, may optionally and additionally comprise amino acid extensions in the C-terminal or N-terminal. In some examples, such variants comprising 3 or more amino acid modifications, such as substitutions relative to SEQ ID NO: 1, may optionally and additionally comprise amino acid extensions in the C-terminal or N-terminal.

In some cases, the claimed variant has no more than 5 modifications, such as substitutions relative to SEQ ID NO:1, suitably no more than 4, no more than 3 or no more than 2 modifications, such as substitutions relative to SEQ ID NO: 1. In some cases, the claimed variant has 1 modification, which is a substitution relative to SEQ ID NO:1. In some cases, the claimed variant has 2 modifications, such as substitutions relative to SEQ ID NO:1. In some cases, the claimed variant has 3 modifications, such as substitutions relative to SEQ ID NO:1. In some cases, the claimed variant has 4 modifications, such as substitutions relative to SEQ ID NO:1. In some cases, the claimed variant has 5 modifications, such as substitutions relative to SEQ ID NO:1.

The amino acid modifications, such as substitutions described herein may comprise the replacement of an amino acid from the hIL-22 sequence with a naturally occurring amino acid (a "natural amino acid") or a synthetic amino acid (a "non-natural amino acid"). In some cases, the substitution(s) comprises the replacement of an amino acid from the hIL-22 sequence with a natural amino acid. In some cases, there are natural amino acids at locations 19, 87 and 94 of the IL-22 variant.

### Substitutions

In some cases, the IL-22 variant can be described as an IL-22 variant comprising at least one amino acid substitution X₁₉, X₈₇ or X₉₄ as defined general formula (I) and further optionally comprising additional amino acid modifications, such as substitutions in other positions than X₁₉, X₈₇ or X₉₄, provided that the sequence identity of the IL-22 variant is between 94% and 99.5% to SEQ ID NO:1.

In some examples such cases provide IL-22 variants with sequence identity of between 94% and 99.5% to SEQ ID NO:1. In some examples such cases provide IL-22 variants with sequence identity of between 94% and 98% to SEQ ID NO:1. In some examples such cases provide IL-22 variants with sequence identity of between 94% and 97% to SEQ ID NO:1.

In some cases, the IL-22 variant can be described as an IL-22 variant comprising at least one amino acid substitution X₁₉, X₈₇ or X₉₄ as defined general formula (I) and further optionally comprising up to 7 additional amino acid modifications, such as substitutions in other positions than X₁₉, X₈₇ or X₉₄, provided that the total number of modifications (e.g. substitutions) relative to SEQ ID NO:1 is between 1 and 8.

In some examples such cases provide IL-22 variants of formula (I) comprising at least two amino acid substitution X₁₉, X₈₇ or X₉₄ as defined general formula (I) and further optionally comprising up to 6 additional amino acid modifications, such as substitutions in other positions than X₁₉, X₈₇ or X₉₄, provided that the total number of modifications (e.g. substitutions) relative to SEQ ID NO:1 is between 1 and 8.

In some examples such cases provide IL-22 variants of formula (I) comprising at least three amino acid substitution X₁₉, X₈₇ or X₉₄ as defined general formula (I) and further optionally comprising up to 5 additional amino acid modifications, such as substitutions in other positions than X₁₉, X₈₇ or X₉₄, provided that the total number of modifications (e.g. substitutions) relative to SEQ ID NO:1 is between 1 and 8.

In some examples such cases provide IL-22 variants of formula (I) comprising up to 7 additional amino acid modifications, such as substitutions in other positions than X₁₉, X₈₇ or X₉₄, provided that the total number of modifications (e.g. substitutions) relative to SEQ ID NO:1 is below 10, such as 9 or 8.

In some examples such cases provide IL-22 variants of formula (I) comprising up to 7 additional amino acid modifications, such as substitutions in other positions than X₁₉, X₈₇ or X₉₄, provided that the total number of modifications (e.g. substitutions) relative to SEQ ID NO:1 is 8 or lower.

In some examples such cases provide IL-22 variants of formula (I) comprising up to 6 additional amino acid modifications, such as substitutions in other positions than X₁₉, X₈₇ or X₉₄, provided that the total number of modifications (e.g. substitutions) relative to SEQ ID NO:1 is between 7 and 8.

In some examples such cases provide IL-22 variants of formula (I) comprising up to 5 additional amino acid modifications, such as substitutions in other positions than X₁₉, X₈₇ or X₉₄, provided that the total number of modifications (e.g. substitutions) relative to SEQ ID NO:1 is 6, 7 or 8.

In some examples such cases provide IL-22 variants of formula (I) comprising up to 4 additional amino acid modifications, such as substitutions in other positions than X₁₉, X₈₇ or X₉₄, provided that the total number of modifications (e.g. substitutions) relative to SEQ ID NO:1 is 5, 6 or 7.

In some examples such cases provide IL-22 variants of formula (I) comprising up to 3 additional amino acid modifications, such as substitutions in other positions than X₁₉, X₈₇ or X₉₄, provided that the total number of modifications (e.g. substitutions) relative to SEQ ID NO:1 is between 1 and 6, between 2 and 6 or between 3 and 6.

In some examples such cases provide IL-22 variants of formula (I) comprising up to 2 additional amino acid modifications, such as substitutions in other positions than X₁₉, X₈₇ or X₉₄, provided that the total number of modifications (e.g. substitutions) relative to SEQ ID NO:1 is 3, 4 or 5.

In some examples such cases provide IL-22 variants of formula (I) comprising up to 1 additional amino acid modifications, such as substitutions in other positions than X₁₉, X₈₇ or X₉₄, provided that the total number of modifications (e.g. substitutions) relative to SEQ ID NO:1 is 2, 3 or 4.

Thus, in some cases a variant of IL-22 is of general formula (I): 
APISSHCRLD KSNFQQPY-X19-T NRTFMLAKEA SLADNNTDVR LIGEKLFHGV
SMSERCYLMK QVLNFTLEEV LFPQSDRFQP YMQEVV-X87-FL-A-RLS-X94-RLSTCH general formula (I)
IEGDDLHIQR NVQKLKDTVK KLGESGEIKA IGELDLLFMS LRNACI
 wherein
a. X₁₉ is I, which is the native amino acid of SEQ ID NO:1 in position 19 or is a non-native hydrophobic amino acid;
b. X₈₇ is P, which is the native amino acid of SEQ ID NO:1 in position 87 or a hydrophobic amino acid or a positively charged amino acid, suitably a hydrophobic amino acid comprising an aromatic ring;
c. X₉₄ is N, which is the native amino acid substitution of SEQ ID NO:1 in position 94 or is a hydrophobic amino acid; and
wherein at least one of said amino acids X₁₉, X₈₇ and X₉₄ is a non-native amino acid relative to SEQ ID NO:1.

In some cases, such variant of IL-22 is of general formula (I) wherein
a. X₁₉ is I, which is the native amino acid of SEQ ID NO:1 in position 19 or is a non-native hydrophobic amino acid;
b. X₈₇ is P, which is the native amino acid of SEQ ID NO:1 in position 87 or a hydrophobic amino acid or a positively charged amino acid, suitably a hydrophobic amino acid comprising an aromatic ring;
c. X₉₄ is N, which is the native amino acid substitution of SEQ ID NO:1 in position 94 or is a hydrophobic amino acid; and
wherein at least one of said amino acids X₁₉, X₈₇ and X₉₄ is a non-native amino acid relative to SEQ ID NO:1; and
with at least one of said amino acids X₁₉, X₈₇ and X₉₄ being a non-native amino acid relative to SEQ ID NO:1;
further optionally comprises up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 has a sequence identity of between 94% and 99.5% to SEQ ID NO:1 and is of general formula (I) and additionally comprises an N-terminal extension. In some cases, a variant of IL-22 has a sequence identity of between 94% and 99.5% to SEQ ID NO:1 and is of general formula (I) and additionally comprises a C-terminal extension.

In some cases, a variant of IL-22 is of general formula (I), wherein if X₁₉ is I, then
a. X₈₇ is not P or X₉₄ is not N; or
b. X₈₇ is not P and X₉₄ is not N; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein if X₁₉ is I, then
a. X₈₇ is a hydrophobic amino acid or a positively charged amino acid, suitably a hydrophobic amino acid comprising an aromatic ring or X₉₄ is a hydrophobic amino acid; or
b. X₈₇ is a hydrophobic amino acid or a positively charged amino acid, suitably a hydrophobic amino acid comprising an aromatic ring and X₉₄ is a hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein if X₁₉ is I, then
a. X₈₇ is a positively charged amino acid or X₉₄ is a hydrophobic amino acid; or
b. X₈₇ is a positively charged amino acid and X₉₄ is a hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein if X₁₉ is I, then
a. X₈₇ is a hydrophobic amino acid or X₉₄ is a hydrophobic amino acid; or
b. X₈₇ is a hydrophobic amino acid and X₉₄ is a hydrophobic amino acid and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein if X₁₉ is I, then
a. X₈₇ is a hydrophobic amino acid comprising an aromatic ring or X₉₄ is a hydrophobic amino acid; or
b. X₈₇ is a hydrophobic amino acid comprising an aromatic ring and X₉₄ is a hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₈₇ is P, then
c. X₁₉ is not I or X₉₄ is not N; or
d. X₁₉ is not I and X₉₄ is not N; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₈₇ is P, then
c. X₁₉ is a hydrophobic amino acid or X₉₄ is a non-native hydrophobic amino acid; or
d. X₁₉ is a hydrophobic amino acid and X₉₄ is a hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₉₄ is N, then
a. X₈₇ is not P or X₁₉ is not I; or
b. X₈₇ is not P and X₁₉ is not I; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₉₄ is N, then
e. X₈₇ is a hydrophobic amino acid or a positively charged amino acid, suitably a hydrophobic amino acid comprising an aromatic ring or X₁₉ is a non-native hydrophobic amino acid; or
f. X₈₇ is a hydrophobic amino acid or a positively charged amino acid, suitably a hydrophobic amino acid comprising an aromatic ring and X₁₉ is a non-native hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₉₄ is N, then
a. X₈₇ is a positively charged amino acid or X₁₉ is a non-native hydrophobic amino acid; or
b. X₈₇ is a hydrophobic amino acid and X₁₉ is a non-native hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₉₄ is N, then
a. X₈₇ is a hydrophobic amino acid or X₁₉ is a non-native hydrophobic amino acid; or
b. X₈₇ is a hydrophobic amino acid and X₁₉ is a non-native hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₉₄ is N, then
a. X₈₇ is a hydrophobic amino acid comprising an aromatic ring or X₁₉ is a non-native hydrophobic amino acid; or
b. X₈₇ is a hydrophobic amino acid comprising an aromatic ring and X₁₉ is a hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein, if X₉₄ is N, then
a. X₈₇ is a hydrophobic amino acid comprising an aromatic ring or X₁₉ is a non-native hydrophobic amino acid; or
b. X₈₇ is a hydrophobic amino acid comprising an aromatic ring and X₁₉ is a non-native hydrophobic amino acid; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; and optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; and optionally comprising up to 6 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; and optionally comprising up to 5 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V and L; and
wherein at least one of said amino acids X₁₉, X₈₇ and X₉₄ is a non-native amino acid relative to SEQ ID NO:1; and optionally comprising up to 4 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; and optionally comprising up to 3 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; and optionally comprising up to 2 additional amino acid modifications, such as substitutions in other positions than X₁₉, X₈₇ or X₉₄, provided that the sequence identity of the IL-22 variant is between 94% and 99.5% to SEQ ID NO:1.

In some cases, a variant of IL-22 is of general formula (I), wherein at least one amino acid substitution is selected from:
a. X₁₉ is I, which is the native amino acid of SEQ ID NO:1 in position 19 or selected from the group consisting of: F, W, A, V and L;
b. X₈₇ is P which is the native amino acid of SEQ ID NO:1 in position 87, or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; and optionally comprising up to 1 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein at least one amino acid substitution is selected from:
a. X₁₉ is F or W;
b. X₈₇ is P or selected from the group consisting of: R, F, H, K and W; and/or
c. X₉₄ is N, F, or W; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein at least one amino acid substitution is selected from:
a. X₁₉ is I or selected from the group consisting of: F W, A, V and L;
b. X₈₇ is selected from the group consisting of: R, F, H, K, W, A, V, I and L; and/or
c. X₉₄ is N or selected from the group consisting of: F, W, A, V, I, and L; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein at least one amino acid substitution is selected from:
a. X₁₉ is I, F or W;
b. X₈₇ is selected from the group consisting of: R, F, H, K and W; and/or
c. X₉₄ is N, F or W; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein at least one amino acid substitution is selected from:
a. X₁₉ is I selected from the group consisting of: F W, A, V and L;
b. X₈₇ is P selected from the group consisting of: R, F, H, K, W, A, V, I and L; and/or
c. X₉₄ is selected from the group consisting of: F, W, A, V, I, and L; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein at least one amino acid substitution is selected from:
a. X₁₉ is I, F or W;
b. X₈₇ is P selected from the group consisting of: R, F, H, K and W; and/or
c. X₉₄ is F or W; and
further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is I, F or W, X₈₇ is P, R or F, and X₉₄ is F or W further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is I or F and/or X₈₇ is R or F and/or X₉₄ is N or F further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is I or F and/or X₈₇ is R or F and/or X₉₄ is N further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is R or F and/or X₉₄ is N or F further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is R or F and/or X₉₄ is N and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is I or F and/or X₈₇ is P and/or X₉₄ is F further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is I or F and/or X₈₇ is P and/or X₉₄ is N and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is P and/or X₉₄ is F further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is P and/or X₉₄ is N and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is R and/or X₉₄ is F and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is R and/or X₉₄ is N and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is R and/or X₉₄ is F and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

One example of a variant of IL-22 is of general formula (I), wherein X₁₉ is F and/or X₈₇ is R and/or X₉₄ is N and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

### Position 19 or X₁₉ of general formula I

In some examples, the substitution at position 19 of SEQ ID NO:1 is a non-conservative substitution. In some examples, the amino acid Isoleucine (I) of position 19 of SEQ ID NO:1 is substituted with a hydrophobic amino acid comprising an aromatic ring. In some examples, the amino acid Isoleucine (I) of position 19 of SEQ ID NO:1 is substituted with a hydrophobic amino acid comprising an aromatic ring. In some such cases, the substituted amino acid is a natural amino acid. In some cases, the amino acid Isoleucine (I) of position 19 of SEQ ID NO:1 is substituted with Phenylalanine (F), or Tryptophan (W). Such substitutions may be referred to as I19F and I19W. In some cases, the variant comprises a substitution selected from I19F and I19W, and in some particular examples, the variant comprises an I19F substitution.

In some further examples, the amino acid Isoleucine (I) of position 19 of SEQ ID NO:1 is substituted with Alanine (I19A), Valine (I19V), or Leucine (I19L).

### Position 87 or X₈₇ of general formula I

In some examples, the substitution at position 87 of SEQ ID NO:1 is a non-conservative substitution. In some examples, the amino acid Proline (P) of position 87 of SEQ ID NO:1 is substituted with a positively charged amino acid, suitably a natural positively amino acid. In other words, in such cases, the substitution is selected from P87R, P87H, P87K. In some cases, the amino acid Proline (P) of position 87 of SEQ ID NO:1 is substituted with a positively charged amino acid. In some cases, the positively charged amino acid may be a natural amino acid, and the substitution is selected from P87R, P87H and P87K. In some cases, the substitution is selected from P87R and P87K. In some cases, the variant comprises a P87R substitution.

In some further examples, the amino acid Proline (P) of position 87 of SEQ ID NO:1 is substituted with Alanine (P87A), Valine (I87V), Isoleucine (P87I) or Leucine (P87L). In some further examples, the amino acid Proline (P) of position 87 of SEQ ID NO:1 is substituted with Phenylalanine (P87F) or Tryptophane (W) In some further examples, the amino acid Proline (P) of position 87 of SEQ ID NO:1 is substituted with Phenylalanine (P87F).

### Position 90

In some examples, the substitution at position 90 of SEQ ID NO:1 is a non-conservative substitution. In some examples, the amino acid Alanine of position 90 of SEQ ID NO:1 is substituted with a hydrophobic amino acid comprising an aromatic ring. In some such cases, the substituted amino acid is a natural amino acid. In other words, in such cases, the amino acid Alanine of position 90 of SEQ ID NO:1 is substituted with Phenylalanine (F) or Tryptophan (W). In other words, in such cases, the substitution is selected from A90F, A90W.

### Position 94 or X₉₄ of general formula I

In some examples, the substitution at position 94 of SEQ ID NO:1 is a non-conservative substitution. In some examples, the amino acid Asparagine (N) of position 94 of SEQ ID NO:1 is substituted with a hydrophobic amino acid, suitably a hydrophobic amino acid comprising an aromatic ring. In some instances, the amino acid Asparagine (N) of position 94 of SEQ ID NO:1 is substituted with a hydrophobic amino acid comprising an aromatic ring. In some cases, the amino acid Asparagine (N) of position 94 of SEQ ID NO:1 is substituted with a natural amino acid. In some cases, the substitution at position 94 is selected from, N94For N94W; and preferably N94F. In some cases, the variant comprises an N94F substitution.

In some cases, the amino acid Asparagine (N) of position 94 of SEQ ID NO:1 is substituted with an Alanine (N94A), Valine (N94A), Isoleucine (N94I) or Leucine (N94L).

### Combinations

In some examples, the IL-22 variant described herein includes further amino acid substitutions. In some cases, the variant further comprises at least one amino acid substitution selected from S12E, N13A, Q83A, E84A, R91A or R95A. In some cases, it further comprises at least one amino acid substitution selected from A1G, A1H, N35D, N35H, N35Q, N64D, N64Q, N64W, A90F, Q113R and K114R, suitably at least one amino acid substitution selected from N35Q and N64Q.

In some examples, the variant includes at least two amino acid substitutions selected from: (i) I19F and P87R; (ii) I19F and N94F; (iii) I19F and S12E; (iv) I19F and N13A; (v) I19F and Q83A; (vi) I19F and E84A; (vii) I19F and R91A; (viii) I19F and R95A; (ix) I19F and A1G; (x) I19F and A1H; (xi) I19F and N35D; (xii) I19F and N35H; (xiii) I19F and N35Q; (xiv) I19F and N64D; (xv) I19F and N64Q; (xvi) I19F and N64W; (xvii) I19F and Q113R; (xviii) I19F and K114R; and (xix) I19F and A90F. In some examples, the variant comprises a non-native hydrophobic amino acid in position 19. In some examples, the variant comprises an amino acid in position 19, which is a non-native hydrophobic amino acid comprising an aromatic ring.

To the extent that the substitution is not already present, each of the preceding options may additionally comprise (a) P87R and/or (b) N94F.
In some examples, the variant includes at least two amino acid substitutions selected from: (i) P87R and I19F; (ii) P87R and N94F; (iii) P87R and S12E; (iv) P87R and N13A; (v) P87R and Q83A; (vi) P87R and E84A; (vii) P87R and R91A; (viii) P87R and R95A; (ix) P87R and A1G; (x) P87R and A1H; (xi) P87R and N35D; (xii) P87R and N35H; (xiii) P87R and N35Q; (xiv) P87R and N64D; (xv) P87R and N64Q; (xvi) P87R and N64W; (xvii) P87R and Q113R; (xviii) P87R and K114R; and (xix) P87R and A90F. In some examples, the variant comprises a hydrophobic amino acid in position 87. In some examples, the variant comprises a positively charged amino acid in position 87. To the extent that the substitution is not already present, each of the preceding options may additionally comprise (a) I19F and/or (b) N94F.

In some examples, the variant includes at least two amino acid substitutions selected from: (i) N94F and I19F; (ii) N94F and P87R; (iii) N94F and S12E; (iv) N94F and N13A; (v) N94F and Q83A; (vi) N94F and E84A; (vii) N94F and R91A; (viii) N94F and R95A; (ix) N94F and A1G; (x) N94F and A1H; ; (xi) N94F and N35D; (xii) N94F and N35H; (xiii) N94F and N35Q; (xiv) N94F and N64D; (xv) N94F and N64Q; (xvi) N94F and N64W; (xvii) N94F and Q113R; (xviii) N94F and K114R; and (xix) N94F and A90F. In some examples, the variant comprises a hydrophobic amino acid in position 94. In some examples, the variant comprises an amino acid in position 94, which is a hydrophobic amino acid comprising an aromatic ring.

To the extent that the substitution is not already present, each of the preceding options may additionally comprise (a) I19F and/or (b) P87R.

In some examples, the variant comprises a hydrophobic amino acid in position, 19, 87 and 94. In some examples, the variant comprises a hydrophobic amino acid in position, 19 and 94 and either a hydrophobic amino acid or a positively charged amino acid in position 87.

The variant of the invention may additionally comprise one or more further substitutions, such as (and to the extent that the following positions are compatible with the preceding embodiments): P2H, I3H, I3V, S4H, S4N, S5H, S5T, H6R, C7G, R8G, R8K, L9S, D10E, D10S, K11G, K11V, N13G, F14S, Q15E, Q16V, P17L, Y18F, I19Q, T20V, R22S, F24H, M25E, M25L, L26S, A27L, E29P, A30Q, L32R, A33N, D34F, N36Q, T37I, D38L, V39Q, R40W, L41Q, I42P, E44R, K45A, F47T, H48G, H48R, G49N, V50S, M52A, M52L, M52V, S53K, S53Y, E54D, E54F, R55Q, R55V, C56Q, L58K, M59I, Q61E, V62D, N64W, F65G, L67Q, E69D, E69L, V70S, F72D, F72L, P73L, Q74T, R77I, F78Q, Q79E, M82Y, Q83G, E84R, V86A, F88N, A90P, A90T, R91K, R91Y, L92R, S93Y, N94Q, R95K, R95Q L96E, S97K, T98N, T98S, C99V, H100S, E102S, G103D, D104Y, D105Y, L106E, L106Q, H107L, H107N, I108L, Q109Y, R110K, N111K, V112E, L115V, K116Y, D117E, T118G, V119A, K120H, L122A, G123V, G126Y, I128V, K129V, G132Y, E133Q, L134P, D135M, L137D, F138R, M139L, M139R, L141Q, N143S, A144E, C145E, I146R and/or I146V. All combinations of substitutions are envisaged and may be present in variants according to the invention provided that there is the required minimum sequence identify with SEQ ID NO:1.

### Other variations

Alternatively, or in addition, the variations within the native sequence may also include amino acid insertions. Up to five, 10, 15, 20, 25, 30, 35, 40, 45 or even up to 50 amino acids may be inserted within the native sequence. In some cases, the insertion may be a trimer, pentamer, septamer, octamer or nonamer for example.

Sequence variations outside of the 146-amino acid sequence of hIL-22, may include an extension, such as the addition of a peptide at the N-terminal end. The peptide may consist of up to five, 10, 15, 20, 25, 30, 35, 40, 45 or even up to 50 amino acids. For example, monomers, trimers, octamers, 13-mers, 15-mers, 16-mers, 21-mers, 27-mers are suitable N-terminus extensions. In some embodiments, the IL-22 variant of the invention may comprise an N-terminal G-P-G* (where * indicates the point of attachment).

Sequence variations outside of the 146-amino acid sequence of hIL-22, may include the addition of a peptide at the C-terminal end. The peptide may consist of up to five, 10, 15, 20, 25, 30, 35, 40, 45 or even up to 50 amino acids.

The variants of the invention may include both an N-terminal and a C-terminal peptide in addition to the variant hIL-22 amino acid sequence as herein described.

The variations within the native sequence may also or alternatively comprise one or more amino acid deletions within SEQ ID NO:1. The variant of the invention may comprise up to five amino acid deletions from SEQ ID NO:1. No more than three or two amino acid deletions are preferred. In such cases the amino acid sequence relative to SEQ ID NO:1 will be between 142-145 in length, however may optionally comprise an amino acid extension in the N- or C-terminal, which may increase the number of amino acids according to the length of said optional N- or C-terminal extension. Said deletions may be present in separate (i.e. non-consecutive) positions, e.g., within SEQ ID NO:1. The variations may also or alternatively be a deletion of two, three, four or five consecutive amino acids within SEQ ID NO:1, meaning that a series of up to five neighbouring amino acids may be deleted.

### Derivatisation

The variants of the invention may be derivatized, wherein the derivatization comprises one or more of (i) a covalently attached fatty acid and (ii) a covalently attached PEG group; or alternatively refers to an Fc-fusion protein, which is a recombinant protein comprising the IL-22 protein and at least a portion of an antibody Fc protein.

As noted above, IL-22 is generally cleared quickly from the body by the kidneys, which limits its use in clinical practice. Known methods for extending the half-life of circulating IL-22 therefore seek to artificially increase the size of IL-22 beyond 70 kDa, to avoid renal clearance. One known solution is the provision of an Fc-fusion protein comprising an IL-22 variant and at least a portion of an antibody Fc protein; Genentech and Evive (previously Generon Shanghai), both have long-acting IL-22-Fc fusions in clinical development. Modifying IL-22 with polyethylene glycol (PEGylation) is another known means for avoiding renal clearance. Alternatively, lipids, such as fatty acids, can be covalently attached to IL-22, which enables binding to albumin, thereby preventing renal excretion and providing some steric protection against proteolysis.

### Calculating Percentage Identity

The skilled technician will appreciate how to calculate the percentage identity between two amino acid sequences. An alignment of the two sequences must first be prepared, followed by calculation of the sequence identity value. The percentage identity for two sequences may take different values depending on: (i) the method used to align the sequences, for example, ClustalW, BLAST, FASTA, Smith-Waterman (implemented in different programs), or structural alignment from 3D comparison; and (ii) the parameters used by the alignment method, for example, local versus global alignment, the pair-score matrix used (for example, BLOSUM62, PAM250, Gonnet etc.) and gap-penalty, for example, functional form and constants.

Having made the alignment, there are various ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of amino acid sequences is a complex process. The popular multiple alignment program ClustalW [48,49] is a preferred way for generating multiple alignments of proteins in accordance with the invention. Suitable parameters for ClustalW may be as follows: For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid sequences may then be calculated from such an alignment as (N/T)*100, where N is the number of positions at which the sequences share an identical residue, and T is the total number of positions compared including gaps but excluding overhangs. Hence, a most preferred method for calculating percentage identity between two sequences comprises (i) preparing a sequence alignment using the ClustalW program using a suitable set of parameters, for example, as set out above; and (ii) inserting the values of N and T into the following formula: Sequence Identity = (N/T)*100.

Alternative methods for identifying similar sequences will be known to those skilled in the art.

Note that C-terminal and N-terminal extensions are disregarded when calculating percentage identity.

### Variant Preparation

The IL-22 variant can be obtained by any means known in the art, including recombinant means. The production of recombinant hIL-22 has been previously described and is well-known in the art. Desired IL-22 variants can be produced in a similar manner. An experienced investigator in the field would be readily able to identify suitable nucleic acid sequences that encode the desired variant IL-22 variants. The skilled person would hence be readily able to execute this part of the invention, based upon the existing knowledge in the art. Suitably, the IL-22 variants are produced in mammalian systems, such as in Chinese hamster ovary (CHO) cells, using standard techniques. A polyhistidine tag (His-tag) may be employed to aid affinity purification of the recombinant variants.

In this regard, IL-22 variants as used in the invention can be prepared using a post expression cleavable His-tag - an N-terminal addition of less than 10, preferably six, histidine residues that can be purified by affinity to a nickel column. The His-tag is linked to the N-terminal of the variant via a linker that can be digested by a known protease to leave the free IL-22 variant. The cleavable His-tag can have the amino acid sequence, HHHHHHDDDDK, whose consensus sequence for the native cut sites is DDDK\, where '\' denotes the cleaved peptide bond site. After nickel purification, the protein was buffer exchanged to 50mM Tris, 150mM NaCl pH8.0 buffer. Cleavage may be achieved by incubating approximately with a mass ratio of 2000:1(protein v.s. protease) at 4°C for overnight.

To further illustrate the invention, a representative process for variant preparation is provided as follows. The process involves preparing a plasmid DNA that encodes the desired amino acid sequence of the IL-22 variant. This plasmid can be transiently transfected into a cell line, for example CHO-K1, which is allowed to grow in a relevant medium before growth is increased by the addition of a known enhancer. The secreted IL-22 variant can then be harvested through known methods of centrifugation (10000g for 40 min) and sterile filtration(0.22uM) before the variant is purified on a nickel column (Cytiva). Then the protein was buffer exchanged into 50mM Tris, 150mM NaCl pH8.0 using Amicon^{®} Ultra. Then a homemade Enterokinase was used to digest the protein with a mass ratio of 2000:1 (Protein v.s. protease) at 4 centi-degree for overnight. After that the protein was reloaded onto a nickel column to collect the flow through (to remove undigested protein), finally the protein was loaded onto a Superdex75 SEC(Cytiva) column to remove aggregates with 1*PBS pH7.4 as mobile phase. Concentration is detected using A280 (NanoDropOne), analysis of the final product using SDS-PAGE, size exclusion chromatography or liquid chromatography with tandem mass spectrometry (LC-MS-MS) with, or without, de-glycosylation can be used to ensure the quality of the final product.

### Identifying Tissue Specificity

As noted above, tissue selectivity means that the response induced by that the IL-22 variant is biased, inducing a greater response in one tissue type over another. Typically, for a pharmaceutically relevant bias, the bias is larger than the bias for wild-type hIL-22. Tissue selectivity may arise from (a) different Eₘₐₓ values in the respective tissues, or (b) different potencies in the respective tissues, such that at a given drug concentration, a greater response is induced in one tissue over another.

Identifying tissue selectivity involves consideration of Eₘₐₓ and EC₅₀ values for the drug in the respective tissue cell lines. Eₘₐₓ and EC₅₀ values can be determined by assays known in the art. Some such assays are described in detail in the Examples section below. When comparing Eₘₐₓ or EC₅₀ values for a variant and for hIL-22, it is only important that the same methodology be used to determine the values for the variants and native proteins.

A **"wild-type relative Eₘₐₓ"** (% activity of hIL-22) is calculated by dividing the Eₘₐₓ of the variant by the Eₘₐₓ of the hIL-22 control used in the experiment after subtraction of the background signal. In some variants of the invention, the wild-type relative Eₘₐₓ(%) (colon) may be at least 2 times, 3 times, 4 times or 5 times larger than the wild-type relative Eₘₐₓ(%) (skin) and/or the wild-type relative Eₘₐₓ(%) (liver). In some variants of the invention, the wild-type relative Eₘₐₓ(%) (colon) may be at least 5 times larger than the wild-type relative Eₘₐₓ(%) (skin) and/or the wild-type relative Eₘₐₓ(%) (liver). In some variants of the invention, the wild-type relative Eₘₐₓ(%) (colon) may be at least 4 or 5 times larger than the wild-type relative Eₘₐₓ(%) (skin) and/or the wild-type relative Eₘₐₓ(%) (liver). In some variants of the invention, the wild-type relative Eₘₐₓ(%) (colon) may be more than 5 times larger than the wild-type relative Eₘₐₓ(%) (skin) and/or the wild-type relative Eₘₐₓ(%) (liver). Differences in Eₘₐₓ values of such orders of magnitude may be considered to indicate tissue selectivity.

Tissue selectivity can also be achieved even where there are not significant differences between the wild-type relative Eₘₐₓ values for respective tissues. In such cases, tissue selectivity arises from differences in the potency, such that at a given drug concentration, a greater response is induced in one tissue over another. In some variants of the invention, the EC₅₀(skin)/EC₅₀(colon) and/or the EC₅₀(liver)/EC₅₀(colon) values for the variant are larger than the corresponding hIL-22 value. In some cases, the values for the variant may be at least 2 times, 3 times, 4 times or 5 times larger than the values for the hIL-22 reference protein. In some cases, the values for the variant may be more than 5 times larger than the values for the hIL-22 reference protein.

In some cases, the tissue specificity of an IL-22 variant can be achieved by modulating both parameters described above, e.g., reducing the wild-type relative Eₘₐₓ of an IL-22 variant for a tissue, which is not desired to target, while also keeping the potency of the IL-22 variant on receptors in the desired tissues over the undesired tissues.

In some cases, the tissue specificity of an IL-22 variant can be achieved by modulating both parameters described above, e.g., increasing the wild-type relative Eₘₐₓ of an IL-22 variant for a tissue, which is desired to target, while also keeping the potency of the IL-22 variant on receptors in the desired tissues over the undesired tissues.

In some cases, the tissue specificity of an IL-22 variant can be achieved by modulating both parameters described above relative to each other, that the resulting overall efficiency and potency results in an IL-22 variant, that is more active and potent for targeting a desired tissue and less so in others e.g., by compensating a decreased or neutral wild-type relative Eₘₐₓ of an IL-22 variant for a tissue, which is desired to target, with an increased potency of the IL-22 variant on receptors in the desired tissues over the undesired tissues.

In some cases, the tissue specificity of an IL-22 variant can be achieved by modulating both parameters described above relative to each other, that the resulting overall efficiency and potency results in an IL-22 variant, that is more active and potent for targeting a desired tissue and less so in others e.g., by compensating for an IL-22 variants lower potency in a desired target tissue, with an wild-type relative Eₘₐₓ in the same tissue.

### Pharmaceutical Composition

Therefore, according to a further aspect of the invention, there is provided a pharmaceutical composition comprising a variant (or derivative thereof) as described herein and a pharmaceutically acceptable vehicle. The formulation of pharmaceutically active ingredients with various excipients is known in the art.

The pharmaceutical composition may be suitable for administration by inhalation, by injection, orally, or ocularly, optionally wherein the injection is intraperitoneal, intramuscular subcutaneous or intravenous, as further described herein. In one embodiment subcutaneous administration is preferred. In one embodiment muscular administration is preferred. In one embodiment intravenous administration is preferred. In one embodiment oral or rectal administration is preferred.

The pharmaceutical composition comprises an IL-22 variant as described herein. In some cases, an IL-22 variant as described herein is an active pharmaceutical ingredient. In some cases, an IL-22 variant as described herein is the active pharmaceutical ingredient in a pharmaceutical composition as described herein. In some cases, an IL-22 variant as described herein is lipidated and an active pharmaceutical ingredient in a pharmaceutical composition as described herein.

A pharmaceutical composition of invention may comprise any of the different variants of IL-22 (or a derivative thereof) described or envisaged herein.

In one embodiment, the pharmaceutically acceptable vehicle may be a solid; optionally the composition may be in the form of a powder for resuspension. A solid pharmaceutically acceptable vehicle may include one or more substances which may also act as flavouring agents, lubricants, solubilisers, suspending agents, dyes, fillers, glidants, inert binders, preservatives, or dyes. The vehicle may also be an encapsulating material. In powders, the vehicle is a finely divided solid that is in admixture with the finely divided variant according to the invention. The powders preferably contain up to 99% variant. Suitable solid vehicles include, for example calcium phosphate, magnesium stearate, talc, sugars, lactose, dextrin, starch, gelatine, cellulose, and ion exchange resins.

In another embodiment, the pharmaceutical vehicle may be a gel, and the composition may be in the form of a cream or the like.

However, the pharmaceutical vehicle may be a liquid; optionally the pharmaceutical composition is in the form of a solution. Liquid vehicles are used in preparing solutions, suspensions, emulsions, syrups, elixirs, and pressurised compositions. The variant according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilisers, or osmo-regulators. Suitable examples of liquid vehicles for parenteral administration include water (partially containing additives as above, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurised compositions can be a halogenated hydrocarbon or other pharmaceutically acceptable propellant.

The process for preparing a pharmaceutical composition of the invention may thus comprise the usual steps that are standard in the art.

### Use in Therapy

According to a further aspect of the invention, there is provided a variant (or derivative thereof) of the invention or a pharmaceutical composition of the invention, for use in therapy. A method of treating a subject with a variant (or derivative thereof) of the invention, or a pharmaceutical composition comprising the same, is also provided. Any of the different variants of IL-22 described or envisaged herein (including derivatives) are expressly included in these aspects of the invention.

The variant (or derivative thereof) of IL-22 or pharmaceutical composition comprising the same may be administered directly into a subject to be treated.

The variant, derivative or pharmaceutical composition may be administered by any means, including by inhalation, by injection, orally, rectally, or ocularly. When administered by inhalation, it may be via the nose or the mouth. Preferably, the variant (or derivative thereof) or pharmaceutical composition is administered by injection, typically subcutaneously, intramuscular or intravenously. It will be appreciated that providing variants (or derivatives thereof) which do not induce signalling activity in skin tissue is particularly advantageous for subcutaneous administration.

Liquid pharmaceutical compositions, which are sterile solutions or suspensions, can be utilised by, for example, intramuscular, intrathecal, epidural, intraperitoneal and particularly subcutaneous or intravenous injection. The variant may be prepared as a sterile solid composition that may be dissolved or suspended at the time of administration using sterile water, saline or another appropriate sterile injectable medium.

Forms useful for inhalation include sterile solutions, emulsions, and suspensions. Alternatively, the variant may be administered in the form of a fine powder or aerosol via a Dischaler^{®} or Turbohaler^{®}. Nasal inhalations may suitably be in the form of a fine powder or aerosol nasal spray or modified Dischaler^{®} or Turbohaler^{®}.

Oral administration may be suitably via a tablet, a capsule or a liquid suspension or emulsion. Suitable examples of liquid vehicles for oral administration include water (partially containing additives, for example, cellulose derivatives, preferably sodium carboxymethyl cellulose solution), alcohols (including monohydric alcohols and polyhydric alcohols, for example, glycols) and their derivatives, and oils (for example, fractionated coconut oil and arachis oil). The variants of the invention may be administered orally in the form of a sterile solution or suspension containing other solutes or suspending agents (for example, enough saline or glucose to make the solution isotonic), bile salts, acacia, gelatine, sorbitan monoleate, polysorbate 80 (oleate esters of sorbitol and its anhydrides copolymerized with ethylene oxide) and the like. Solutions, syrups, and elixirs also form part of the invention. The variants according to the invention can also be administered orally in solid composition form. Solid compositions suitable for oral administration include pills, capsules, granules, tablets, and powders.

Rectal administration may suitably be via a suppository or foam.

Formulations for ocular administration may be solutions or suspensions for intraocular injection. The variant (or derivative thereof) may be prepared as a sterile solid composition that may be dissolved or suspended at the time of administration using sterile water, saline or other appropriate sterile injectable medium. The formulation may be for subconjunctival, intravitreal, retrobulbar or intracameral injection.

A variant (or derivative thereof) or pharmaceutical composition of the invention may be administered to any subject in need thereof. Most preferably, the subject is a human being. The variants and compositions need not only be administered to those already showing signs of a disease, disorder and/or condition. Rather, they can be administered to apparently healthy subjects as a purely preventative measure against the possibility of such a disease, disorder, and/or condition in future.

It will be appreciated that variants (or derivatives thereof) of IL-22 and compositions according to the invention may be used in a monotherapy (i.e. the sole use of that variant or composition), for treating a disease, disorder, and/or condition. Alternatively, variants (or derivatives thereof) and compositions according to the invention may be used as an adjunct to, or in combination with, known therapies for treating a disease, disorder, and/or condition.

It will be appreciated that the amount of the variant (or derivative thereof) of IL-22 that is required is determined by its biological activity, half-life, and bioavailability, which in turn depends on the mode of administration, the physiochemical properties of the variant and composition, and whether it is being used as a monotherapy or in a combined therapy. The frequency of administration will also be influenced by the half-life of the variant within the subject being treated.

Optimal dosages to be administered may be determined by those skilled in the art and will vary with the particular variant (or derivative thereof) in use, the strength of the pharmaceutical composition, the mode of administration, and the advancement of the disease, disorder and/or condition. Additional factors depending on the particular subject being treated will result in a need to adjust dosages, including subject age, weight, gender, diet and time of administration.

Many studies have demonstrated key effects of IL-22 in multiple epithelial injury models in especially lung, liver, intestine, kidney, skin, pancreas, and thymus. Mechanistically, several pathways within e.g. anti-apoptosis, proliferation, innate immunity, anti-oxidative stress, anti-fibrosis, and stem cell/progenitor cell recruitment have been well documented to meditate IL-22 effects in studies by multiple investigators. Key mechanistic findings have been further confirmed in vitro using human cell lines or in human ex vivo models (e.g. primary human intestinal organoids). The strong role of IL-22 in preventing cell death, securing regeneration, and controlling inflammation in epithelial injury is therefore well established.

The invention also provides a variant, derivative or pharmaceutical composition of the invention for use in a method of treating a metabolic, pulmonary, gut, pancreatic, kidney or CNS disease, disorder and/or condition. Methods of treating a metabolic, pulmonary, gut, pancreatic, kidney or CNS disease, disorder and/or condition using a variant or pharmaceutical composition of the invention are also provided. Any of the different variants of IL-22 described or envisaged herein are expressly included in these aspects of the invention.

The invention also provides for the use of a variant, derivative or pharmaceutical composition of the invention in the manufacture of a medicament for treatment of a metabolic, pulmonary, gut, pancreatic, kidney or CNS disease, disorder and/or condition. Any of the different variants of IL-22 described or envisaged herein are expressly included in these aspects of the invention.

The metabolic disease, disorder and/or condition may be obesity, prediabetes, insulin resistance, diabetes type 1, diabetes type 2, metabolic dysfunction-associated steatohepatitis (MASH), hyperlipidaemia, hyperglycaemia, or hyperinsulinemia.

The pulmonary disease, disorder and/or condition may be COPD, cystic fibrosis, bronchiectasis, idiopathic pulmonary fibrosis, acute respiratory distress syndrome, a chemical injury, a viral infection, a bacterial infection or a fungal infection.

The gut disease, disorder and/or condition may be IBD, ulcerative colitis, Crohn's disease, GvHD, coeliac disease, a chemical injury, a viral infection, or a bacterial infection.

The pancreatic disease, disorder, and/or condition may be an inflammation-related pancreatic disease, disorder, and/or condition. Inflammation-related pancreatic disease, disorder, and/or condition may be acute pancreatitis, non-acute pancreatitis, pancreatic fibrosis, pancreatic carcinoma and diabetes.

The kidney disease, disorder and/or condition may be acute kidney disease or chronic kidney disease.

The CNS disease, disorder and/or condition may be multiple sclerosis.

A method of treating a subject having a condition responsive to IL-22 treatment, such as one or more of the above diseases, disorders, or conditions, with a variant of IL-22 according to the invention, or a pharmaceutical composition comprising the same, is also provided.

There is no restriction on which variant of IL-22, or derivative or pharmaceutical composition as described herein should be administered to which patient (subject). Rather, it is intended that any of the variants according to the invention (and derivatives) and compositions containing the same as described herein can be administered to any patient (subject) as described herein.

All the features described herein (including any accompanying claims, abstract and drawings), and/or all the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made to the Examples, which are not intended to limit the invention in any way.

### EXAMPLES

The material and methods employed in experiments disclosed in the following examples are as follows unless explicitly indicated otherwise.

### Compounds used in the examples

Table 1 provides an overview of the IL-22 compounds tested in the experimental setup described in the EXAMPLES of this application. Compound 1 is human IL-22, also referred to as wild-type IL-22, with the amino acid sequence referred to herein with the sequence identifier: SEQ ID NO:1. The remaining compounds comprise at least one amino acid modification (substitution) relative to SEQ ID NO: 1 and are thus modified human IL-22 variants. Compound 7 comprises two amino acid modifications (substitutions) relative to SEQ ID NO:1.

**Table 1: Compounds used in the examples. Compound 1 comprising SEQ ID NO:1 is the wild-type (wt) human IL-22 protein, compounds 2-7 are all modified IL-22 variants. Compounds 2-6 comprise single amino modifications relative to human IL-22 (SEQ ID NO: 1). Compound 7 comprises two amino modifications relative to human IL-22 (SEQ ID NO: 1).**

| **Compound ID** | **SEQ ID NO:** | **Variation relative to SEQ ID NO: 1** | **Description** |
|---|---|---|---|
| Compound 1 | 1 | None | Wild-type (wt) |
| Compound 2 | 2 | P87R | Modified IL-22 variant |
| Compound 3 | 3 | I19F | Modified IL-22 variant |
| Compound 4 | 4 | P87F | Modified IL-22 variant |
| Compound 5 | 5 | A90F | Modified IL-22 variant |
| Compound 6 | 6 | N94F | Modified IL-22 variant |
| Compound 7 | 7 | P87R, A90F | Modified IL-22 variant |

### Methods

The inventors tested the above-mentioned compound's *in vitro* efficacy (Emax) and potency (EC50) in liver epithelial cells (HepG2 cell line), colon epithelial cells (HT-29 cell line) and a keratinocyte cells (HaCaT cell line) by measuring its phosphorylation of signal transducer and activator of transcription (pSTAT)3 in each cell line.

To perform such test the following assays were applied to:
- Cultivate each cell line before the test, according to Assay I, II and III, for colon cells, skin cells and liver cells, respectively.
- Prepare the compounds for the experiment as described in Assay IV.
- Stimulate the cell lines *in vitro* with the compound indicated and as described in Assay V.
- Quantify the STAT3 phosphorylation after stimulation with the indicated compound and hIL-22 controls.

### Assay I: Human colon cell line (HT-29) culture

The cryopreserved HT-29 cells (cat. no. ATCC-HTB-38) were thawed and cultured in a modified general purpose medium McCoy's 5A (Modified) Medium (McCoy's) plus GlutaMax (cat. no. 36600021, Gibco, Denmark) with 100 U/ml penicillin and 100 mg/ml streptomycin (cat. no. P4333, Sigma-Aldrich, Saint Louis) and 10 % heat-inactivated fetal bovine serum (cat. no. F6765, Sigma-Aldrich) and grown at 37 °C 5% CO₂.

The HT-29 cells were passaged when confluence was above 80 - 90 % using Trypsin-EDTA (cat. no. 25200056, Thermo-Fischer Scientific).

### Assay II: Human skin cell line (HaCaT) culture

Cryopreserved HaCaT cells were thawed and cultured in Dulbecco's Modified Eagles Medium (DMEM) + GlutaMax with 100 U/ml penicillin and 100 mg/ml streptomycin (cat. no. P4333, Sigma-Aldrich, Saint Louis) and 10 % heat-inactivated fetal bovine serum (cat. no. F6765, Sigma-Aldrich) and grown at 37 °C 5% CO₂. The HaCaT cells were passaged when confluence was close to 100 % using Trypsin-EDTA (cat. no. 25200056, Thermo-Fischer Scientific).

### Assay III: Human liver cell line (HepG2) culture

Cryopreserved HepG2 vials were thawed and cultured in Dulbecco's Modified Eagles Medium (DMEM) + GlutaMax (cat. no. 10566-016, Gibco, Denmark) with 100 U/ml penicillin and 100 mg/ml streptomycin (cat. no. P4333, Sigma-Aldrich, Saint Louis, USA) and 10 % heat-inactivated fetal bovine serum (cat. no. F6765, Sigma-Aldrich) and grown at 37 °C 5% CO₂. The HepG2 cells were passaged when confluency was above 70 - 80 % using Trypsin-EDTA (cat. no. 25200056, Thermo-Fischer Scientific).

### Assay IV: Preparing compound for testing in vitro efficacy (Eₘₐₓ) and potency (EC₅₀) in colon, skin and liver cell lines.

All compounds were thawed and diluted in phosphate buffered saline (cat. no. 14190-144, Gibco, Life Technologies, Netherlands) to a concentration of 100 µg/ml (rhIL-22) or 504 µg/ml (IL-22 analogues) under sterile conditions.

Aliquots were stored at -80°C until use. A new aliquot was thawed for each experiment.

Before stimulation of the cell cultures, an aliquot was thawed and diluted into a serum-free medium containing 100 U/ml penicillin and 100 mg/ml streptomycin (1% P/S) pre-heated to 37°C.

### Assay V: In vitro stimulation of colon, skin and liver cells

In preparation for stimulation, the culture media was removed, and 50 µl serum-free media (1% P/S) was added to each well for one hour of cell starvation at 37 °C 5% CO_{2.}

To stimulate the cells, an additional 50 µl serum-free media (1% P/S) containing one of the indicated compounds to each well.
- **The stimulation of HT-29 cells** was performed between passages 3 and 12 (harvested and reseeded) at a seeding density of 70,000 cells/well after 24 hours of adaptation in a 96-well cell culture plate.
- **The stimulation of HaCaT cells** was performed between passages 3 and 8 (harvested and reseeded) at a seeding density of 50,000 cells/well after 24 hours of adaptation in a 96-well cell culture plate.
- **The stimulation of HepG2 cells** was performed between passages 3 and 12 (harvested and reseeded) at a seeding density of 60,000 cells/well after 24 hours of adaptation in a 96-well culture plate.

The culture plate was placed on a plate shaker to mix for 1-2 minutes shortly after the stimulation-step was completed, followed by 15 minutes incubation- step at 37 °C 5% CO_{2.}

Stimulations were always performed in triplicates.

Each experimental setup included two control stimulations, performed at the same time as the stimulation with the test-compound and were also run in triplicate:
- 50 µl serum-free media (1% P/S) and human IL-22 (compound 1)
- 50 µl serum-free media (1% P/S) in triplicate and containing one of the indicated compounds.

### Assay VI: Quantification of STAT3 phosphorylation

The phosphorylation of STAT3 was measured using Alpha SureFire Ultra STAT3 pY507 (cat. no. ALSU-PST3-A500, Perkin Elmer, Denmark) according to the manufacturer's instructions and is briefly described below:
1. Lysis buffer was diluted to the working concentration in ultra-filtrated water.
2. Stimulation medium was removed and 50 µl of lysis buffer was added to each well.
3. The plate was placed on a plate shaker at 300 rpm for 10 minutes and lysates were manually harvested and assayed immediately.
4. 5 µl of Acceptor mix (comprising reaction buffer, activation buffer and AlphaSreen^{®} Acceptor beads) was added to each well of a 348-well alpha plate (cat. no. 6005350, Perkin Elmer, Denmark), followed by 10 µl of the lysates collected under step 3.
5. The plate was centrifuged at 300 x g at 20°C for 2 minutes and incubated at room temperature in the dark for one hour.
6. After completion of step 5, 5 µl of Donor mix (comprising dilution buffer, AlphaSreen^{®} Donor Beads) was added to each well, and centrifugation and one-hour incubation were repeated.
7. The plate was analysed immediately on an EnVision instrument (Perkin Elmer, Denmark) using the AlphaScreen^{®} Label with a 570/100 filter with 'crosstalk correction' disabled.
8. The phosphorylation of STAT3 was reported in alpha-signal counts.
9. The intra-assay coefficient of variance was a median 4%.

### Assay VII: Data analysis and statistics

The raw data was analysed in R Statistical Software (v4.3.2; R Core Team 2021) using the 'drc' package (v3.0.1; Ritz et al 2015).

Individual concentration-response curves were fitted to rhIL-22 and all analogues using a four-parameter non-linear regression with a hill slope constraint of 1 and a lower asymptote (Eₘᵢₙ) fixed to the minimum alpha-signal count value observed in the wells containing only serum-free media (1% P/S).

The EC₅₀ and Eₘₐₓ were estimated from these curves. EC₅₀ was considered not estimated for analogues where the estimated EC value fell outside the tested concentration range or where the Eₘₐₓ was less than 25% of the rhIL-22 control.

For compounds where EC₅₀ could not be estimated, Eₘₐₓ was set to the value at highest concentration where the best fit was a straight line or otherwise also considered not estimated.

The wild-type relative Eₘₐₓ (% activity of rhIL-22 (compound 1)) was calculated by dividing the Eₘₐₓ of the analogue by the Eₘₐₓ of the rhlL-22 control used in the experiment after subtraction of the background signal.

### Example 1 - In vitro efficacy (Eₘₐₓ) and potency (EC₅₀) of the wild-type human IL-22 protein (SEQ ID NO:1) in colon (HT-29), skin (HaCaT) and liver (HepG2) cell lines.

Wild-type human IL-22 (compound 1 - hIL-22) was included as a control in each experiment and thus provided an excellent pool of data to establish a baseline for the efficacy and potency of human IL-22 (compound 1) in each tested cell line (colon (HT-29), skin (HaCaT) and liver (HepG2)).

This baseline shows whether IL-22 as such may present with a biased efficacy and potency in one or more of these tissues' epithelial cells.

The (colon (HT-29), skin (HaCaT) and liver (HepG2) cell lines were cultured according to Assays I, II and III, respectively. Compound 1 (wild-type human IL-22 protein) was prepared according to Assay IV and then used for the stimulation of the cultured cells as described in Assay V. The Quantification and Data analysis was performed according to Assays VI and VII.

### Results - human IL-22 has a bias towards the colon cell line relative to the liver and skin cell lines.

Tables 2 and 3, as well as corresponding Figure 1, show the results of testing compound 1 (wild-type human IL-22 protein) in the three cell lines, representing colon, skin and liver epithelial cells, respectively.

### Efficacy (Eₘₐₓ)

From the Eₘₐₓ values, shown in Table 2, for each cell line after stimulation with compound 1 (wild-type human IL-22 protein) it is evident that the assays are broadly comparable, with somewhat greater variability observed in the liver (HepG2 cell line) compared to the colon (HT-29 cell line) and skin (HaCaT cell line).

**Table 2: Eₘₐₓ (alpha-signal counts) in HT-29 (colon cell line), HaCaT (skin cell line) and HepG2 (liver cell line), #Data presented as median (IQR)**

| **Compound ID** | **SEQ ID NO:** | **Eₘₐₓ in HT-29 (alpha-signal counts)** | **Eₘₐₓ in HaCaT (alpha-signal counts)** | **Eₘₐₓ in HepG2 (alpha-signal counts)** |
|---|---|---|---|---|
| 1 | 1 | 441 (303-504)^{#} | 453 (378-625)^{#} | 702 (519-892)^{#} |

### Potency (EC₅₀)

From the EC₅₀ values, shown in Table 3, for each cell line after stimulation with compound 1, it is evident that compound 1 has greater potency in the colon (HT-29 cell line) compared to the liver (HepG2 cell line) and skin (HaCaT cell line).

**Table 3: EC₅₀ in HT-29 (colon cell line), HaCaT (skin cell line) and HepG2 (liver cell line), *Data is presented as a geometric mean (range).**

| **Compound ID** | **SEQ ID NO:** | **EC₅₀ in HT-29 (µM)** | **EC₅₀ in HaCaT (µM)** | **EC₅₀ in HepG2 (µM)** |
|---|---|---|---|---|
| 1 | 1 | 1.8e-03 (6.9e-04 - 3.3e-03)* | 1.1e-02 (5.4e-03 - 2.1e-02)* | 1.6e-02 (6.8e-03 - 4.8e-02)* |

### Conclusion

Based on the Eₘₐₓ and EC₅₀ values that wild-type human IL-22 protein (compound 1) elicits in this experiment, it can be concluded that IL-22 has a slightly greater potency on colon tissue (HT-29 cell line) than skin (HaCaT cell line) and liver (HepG2 cell line) tissues.

### Example 2 - In vitro efficacy (Eₘₐₓ) and potency (EC₅₀) in colon (HT-29), skin (HaCaT) and liver (HepG2) cell lines, of modified IL-22 variants comprising an amino acid modification P87R.

Cell lines were cultured according to Assays I, II and III, respectively, depending in the cell line, the compounds as indicated were prepared for testing according to Assay IV and then used for the stimulation-step of the cultured cells as described in Assay V. The Quantification and Data analysis was performed according to Assays VI and VII.

### Results - IL-22 variants comprising a P87R modification have greater efficacy and potency in the colon cell line than in the skin and liver cell lines.

The below tables and corresponding Figures 2-5 show the results of testing of the indicated compounds 1, 2, 4, 5 and 7 in the three cell lines, representing colon, skin and liver epithelial cells, respectively.

### Efficacy (Eₘₐₓ)

The Eₘₐₓ and wild-type relative Eₘₐₓ of each cell line after stimulation with the indicated compounds can be reviewed in Table 4A and Table 4B, respectively.

**Table 4A: Eₘₐₓ (alpha-signal counts) of compounds 1 (wild-type human IL-22 protein), 2 (P87R modified IL-22 variant), 4 (P87F modified IL-22 variant), 5 (A90F modified IL-22 variant) and 7 (P87R and A90F modified IL-22 variant), in HT-29 (colon cell line), HaCaT (skin cell line) and HepG2 (liver cell line). #Data presented as median (IQR).**

| **Tested compounds** | | | **Eₘₐₓ (alpha-signal counts)** | | |
|---|---|---|---|---|---|
| **Compound ID** | **SEQ ID NO:** | **Modification relative to SEQ ID NO: 1** | **in HT-29** | **in HaCaT** | **in HepG2** |
| 1 | 1 | None | 441 (303-504)^{#} | 453 (378-625)^{#} | 702 (519-892)^{#} |
| 2 | 2 | P87R | 406 | 82 | 87 |
| 4 | 4 | P87F | 380 | 601 | 466 |
| 5 | 5 | A90F | 510 | 460 | 557 |
| 7 | 7 | P87R, A90F | 303 | 117 | 110 |

**Table 4B: Relative Eₘₐₓ of compounds 1 (wild-type human IL-22 protein), 2 (P87R modified IL-22 variant), 4 (P87F modified IL-22 variant), 5 (A90F modified IL-22 variant) and 7 (P87R and A90F modified IL-22 variant) in HT-29 (colon cell line), HaCaT (skin cell line) and HepG2 (liver cell line).**

| **Tested compounds** | | | **hIL-22 relative Eₘₐₓ (%)** (Eₘₐₓ as percentage of corresponding wild-type human IL-22 protein (compound 1) value) | | |
|---|---|---|---|---|---|
| **Compound ID** | **SEQ ID NO:** | **Modification relative to SEQ ID NO: 1** | **in HT-29** | **in HaCaT** | **in HepG2** |
| 2 | 2 | P87R | 76 | **15** | **3** |
| 4 | 4 | P87F | 123 | 67 | 62 |
| 5 | 5 | A90F | 103 | 95 | 76 |
| 7 | 7 | P87R, A90F | 56 | **10** | **17** |

### Wild-type human IL-22 protein (compound 1) relative Eₘₐₓ

Tissue specificity of a compound can be derived by a smaller Eₘₐₓ in some tissues versus another - in the current context this would be a smaller Eₘₐₓ in the liver (HepG2 cell line) and skin (HaCaT cell line) compared to the colon (HT-29 cell line). For an increase in tissue selectivity relative to compound 1, there is a proportionally greater reduction in the Eₘₐₓ liver and skin values relative to compound 1, as compared to the colon value.

From the wild-type relative Eₘₐₓ values for each cell line after stimulation with compound 2, it is evident that that the P87R modification in human IL-22 (compound 2) has a pronounced effect on the tissue specificity with a close to normal efficacy in the colon (HT-29 cell line) and markedly reduced efficacy in the liver (HepG2 cell line) and skin (HaCaT cell line). Specifically, the wild-type relative Eₘₐₓ value is 5 times higher in the colon cell line relative to the skin cell line and 25 times higher in the colon cell line relative to the liver cell line (Table 4B). Absolute Eₘₐₓ values observed for compound 2 in the skin and liver cell lines are also outside the normal range observed across wild-type experiments in the respective tissues (Table 4A).

Looking at compound 4, the P87F substitution provides a similar, though less pronounced, bias compared to the efficacy of the P87R modification. Specifically, the wild-type relative Eₘₐₓ value is 1.8 times higher in the colon cell line relative to the skin cell line and 2 times higher in colon cell line relative to the liver cell line. The less pronounced efficacy of P87F compared to P87R can also be seen in the absolute Eₘₐₓ values, which for the P87F modification are within the normal range for the wild-type human IL-22 protein (compound 1) in the colon (HT-29 cell line) and skin (HaCaT cell line) but outside the normal range in the liver (HepG2 cell line).

Compound 5 has wild-type relative Eₘₐₓ values without pronounced bias towards any of the tested tissues. Specifically, the wild-type relative Eₘₐₓ value is 1.1 times higher in the colon cell line relative to the skin cell line and 1.4 times higher in the colon cell line versus the liver cell line.

Compound 7, with P87R and A90F modifications, shows a higher efficacy in the colon (HT-29 cell line) compared to the liver (HepG2 cell line) and skin (HaCaT cell line). Specifically, the wild-type relative Eₘₐₓ value is 5.6 times higher in the colon cell line relative to the skin cell line and 3.2 times higher in the colon cell line relative to the liver cell line.

From these results it can be deduced that the P87R modification can reduce the wild-type relative Eₘₐₓ values in skin and liver and thus provides a more colon-selective activity than the wild-type human IL-22 protein (compound 1). Compounds having the P87R modification show greater tissue selectivity than compounds comprising a different modification at P87 (i.e. P87F/compound 4) and/or A90F.

Furthermore, it can be deduced that the P87R modification also provides colon-specific activity when it is combined with more neutral amino acid modifications, such as the A90F modification.

### Potency (EC₅₀)

The EC₅₀ of each cell line after stimulation with the indicated compounds can be reviewed in Table 5A. For compounds 2 and 7, the dose-response curve for skin and liver cell lines did not increase over the tested concentration range. Hence, no meaningful EC₅₀ value could be established, further supporting the conclusion that Compounds 2 and 7 are not functionally efficacious in the colon, liver and skin tissues.

**Table 5A: EC₅₀ of compounds 1 (wild-type human IL-22 protein), 2 (P87R modified IL-22 variant), 4 (P87F modified IL-22 variant), 5 (A90F modified IL-22 variant) and 7 (P87R and A90F modified IL-22 variant), in HT-29 (colon cell line), HaCaT (skin cell line) and HepG2 (liver cell line), *Data presented as geometric mean (range); NE - not estimated, because no meaningful EC₅₀ value could be established.**

| **Tested compounds** | | | **EC₅₀ (µM)** | | |
|---|---|---|---|---|---|
| **Compound ID** | **SEQ ID NO:** | **Modification relative to SEQ ID NO: 1** | **in HT-29** | **in HaCaT** | **in HepG2** |
| 1 | 1 | None | 1.8e-03 (6.9e-04 - 3.3e-03)* | 1.1e-02 (5.4e-03 - 2.1e-02)* | 1.6e-02 (6.8e-03 - 4.8e-02)* |
| 2 | 2 | P87R | 1.6E-02 | NE | NE |
| 4 | 4 | P87F | 3.2E-03 | 1.3E-02 | 4.3E-02 |
| 5 | 5 | A90F | 1.8E-03 | 2.1E-02 | 1.8E-02 |
| 7 | 7 | P87R, A90F | 1.0E-02 | NE | NE |

### Potency (EC₅₀)- skin/colon and liver/colon - ratios

Tissue specificity of a compound can also be derived by a relatively lower potency (higher EC₅₀) in some tissues versus another - in the current context this would be a higher EC₅₀ in the liver (HepG2 cell line) and skin (HaCaT cell line) compared to the colon (HT-29 cell line). For an increase in tissue selectivity relative to compound 1, the difference between the potencies in various cell lines is increased; in the current context, this would mean that the ratios EC₅₀(skin)/EC₅₀(colon) and/or the EC₅₀(liver)/EC₅₀(colon) value(s) is/are larger for the tested variant that for compound 1.

The relative potencies of each of the tested compounds in skin/colon (HaCaT/HT-29 cell lines) and liver/colon (HepG2/HT-29 cell lines), are shown in the Table 5B. Since no meaningful EC₅₀ values could be calculated for compound 2 and compound 7, the ratios for these compounds could likewise not be calculated.

According to Table 5B, compound 1 (human IL-22) is more potent in the colon (HT-29 cell line), than the skin (HaCaT cell line) and liver (HepG2 cell line), as the ratio values are greater than 1. Compound 4 shows a slightly increased potency for skin cell lines relative to colon cell lines. However, broadly speaking, compounds 4 and 5 show a similar bias to compound 1 (human IL-22) in terms of potency towards colon cell lines relative to skin and liver cell lines.

**Table 5B: EC₅₀ of compounds 1 (wild-type human IL-22 protein), 2 (P87R modified IL-22 variant), 4 (P87F modified IL-22 variant), 5 (A90F modified IL-22 variant) and 7 (P87R and A90F modified IL-22 variant), in HT-29 (colon cell line), HaCaT (skin cell line) and HepG2 (liver cell line), *Data presented as a geometric mean (range); NE - not estimated, because no meaningful EC50 value could be established due to the very low Eₘₐₓ.**

| **Tested compounds** | | | **EC₅₀ ratios** | |
|---|---|---|---|---|
| **Compound ID** | **SEQ ID NO:** | **Modification relative to SEQ ID NO: 1** | **HaCaT/HT-29** | **HepG2/HT-29** |
| 1 | 1 | None | 8.3 (4.3-16.6)^{#} | 6.4 (3.6-10.3)^{#} |
| 2 | 2 | P87R | NA | NA |
| 4 | 4 | P87F | 4.1 | 13.4 |
| 5 | 5 | A90F | 11.7 | 10.0 |
| 7 | 7 | P87R, A90F | NA | NA |

### Conclusion

Looking at the above data, it can be seen that compound 2 (P87R modification) is a potent modified IL-22 variant acting as a pronounced colon-specific agonist, with a 5 times higher *wild-type relative Eₘₐₓ value* in colon vs. skin and 25 times higher *wild-type relative Eₘₐₓ value* in colon vs. liver.

Compound 7 underlines the powerful effect that the P87R modification has on the tissue-selective characteristics of modified IL-22 variants comprising P87R, because it also acts as a potent modified IL-22 variant acting as a pronounced colon-specific agonist, with a 5.6 times higher *wild-type relative Eₘₐₓ value* in the colon cell line relative to the skin cell line and 3.2 times higher *wild-type relative Eₘₐₓ value* in the colon cell line relative to the liver cell line, even when combined with a relatively neutral (non-tissue-specific) modification A90F.

Further, the modification P87F presents with *wild-type relative Eₘₐₓ values* that are approximately 2-fold higher in the colon cell lines compared to the skin and liver cell lines. However, the measured Eₘₐₓ values are still within the normal range of human IL-22 for skin (380 of 303-504 normal range for human IL-22) and about 95% of the lowest range value for liver (466 of 492-923 normal range for human IL-22). Therefore, compounds comprising this modification will likely act as less colon-selective agonists compared to compounds comprising P87R, within a given dosage range, because they maintain some agonist activity in the liver and skin.

This example underlines the pronounced effects of the modification P87R in modified IL-22 variants. Compounds with this modification can be used to develop colon-selective variant drugs due to their potent effects in colon and none or very limited effects in skin and liver, skin being the tissue which currently present with the most unwanted effects in clinical trials of IL-22 variant drug candidates.

### Example 3 - In vitro efficacy (Eₘₐₓ) and potency (EC₅₀) in colon (HT-29), skin (HaCaT) and liver (HepG2) cell lines, of modified IL-22 variants comprising a 119F modification

Cell lines where cultures according to Assays I, II and III, respectively, depending in the cell line, the compounds as indicated were prepared for testing according to Assay IV and then used for the stimulation-step of the cultured cells as described in Assay V. The Quantification and Data analysis was performed according to Assays VI and VII.

### Results - IL-22 variants comprising a 119F modification are more efficacious and potent in colon than in skin and liver.

Tables 6A and 6B and corresponding Figure 6 show the results of testing of the indicated compounds 1 and 3 in the colon, skin and liver cell lines, respectively.

### Efficacy (Eₘₐₓ)

The Eₘₐₓ of each cell line after stimulation with the indicated compounds can be reviewed in Table 6A. Compound 3 presents with Eₘₐₓ values within the range of human IL-22 (compound 1), in the colon (HT-29 cell line) and in the skin (HaCaT-cell line), yet an Eₘₐₓ value below the lower range in the liver (HepG2 cell line) - i.e. partial agonism in liver cell line.

**Table 6A: Eₘₐₓ (alpha-signal counts) of compounds 1 (wild-type human IL-22 protein), and compound 3 (N94F modified IL-22 variant), in HT-29 (colon cell line), HaCaT (skin cell line) and HepG2 (liver cell line). #Data presented as a median (IQR).**

| **Tested compounds** | | | **Eₘₐₓ (alpha-signal counts)** | | |
|---|---|---|---|---|---|
| **Compound ID** | **SEQ ID NO:** | **Modification relative to SEQ ID NO: 1** | **in HT-29** | **in HaCaT** | **in HepG2** |
| 1 | 1 | None | 441 (303-504)^{#} | 453 (378-625)^{#} | 702 (519-892)^{#} |
| 3 | 3 | I19F | 338 | 591 | 357 |

### Wild-type human IL-22 protein (compound 1) relative Eₘₐₓ

As shown in Table 6B, the I19F substitution results in neutral *wild-type relative Eₘₐₓ values* with a noticeable reduction in efficacy in HepG2 cells relative to human IL-22, as well as HaCaT cells. Specifically, the wild-type relative Eₘₐₓ value is 1.6 times higher in the colon cell line with respect to the skin cell line and 2.3 times higher in the colon with respect to the liver cell line.

**Table 6B: Relative Eₘₐₓ of compound 1 (wild-type human IL-22 protein), and compound 3 (N94F modified IL-22 variant), in HT-29 (colon cell line), HaCaT (skin cell line) and HepG2 (liver cell line).**

| **Tested compounds** | | | **hIL-22 relative Eₘₐₓ (%)** (Eₘₐₓ as percentage of corresponding wild-type human IL-22 protein (compound 1) value) | | |
|---|---|---|---|---|---|
| **Compound ID** | **SEQ ID NO:** | **Modification relative to SEQ ID NO: 1** | **in HT-29** | **in HaCaT** | **in HepG2** |
| 3 | 3 | I19F | 108 | 66 | 45 |

### Potency (EC₅₀)

As shown in Table 7A, compound 3 (I19F modification) presents within the lower normal range of the human IL-22 reference for EC₅₀ values for colon (HT-29 cell line) and higher end of the normal range in the other two tissues (HaCaT and HepG2 cell lines).

**Table 7A: EC₅₀ of compound 1 (wild-type human IL-22 protein), and compound 3 (N94F modified IL-22 protein), in HT-29 (colon cell line), HaCaT (skin cell line) and HepG2 (liver cell line), *Data presented as geometric mean (range); NE - not estimated, because no meaningful EC₅₀ value could be established due to the very low Eₘₐₓ.**

| **Tested compounds** | | | **EC₅₀ (µM)** | | |
|---|---|---|---|---|---|
| **Compound ID** | **SEQ ID NO:** | **Modification relative to SEQ ID NO: 1** | **in HT-29** | **HaCaT** | **HepG2** |
| 1 | 1 | None | 1.8e-03 (6.9e-04 - 3.3e-03)* | 1.1e-02 (5.4e-03 - 2.1e-02)* | 1.6e-02 (6.8e-03 - 4.8e-02)* |
| 3 | 3 | I19F | 9.7E-04 | 2.1E-02 | 2.7E-02 |

### Potency (EC₅₀)- skin/colon and liver/colon - ratios

The tissue-relative EC₅₀ ratios for compound 3 (I19F modification) shown in Table 7B reveal a 2.6-fold increase in EC₅₀-ratio for HaCaT/HT-29 and 4.3-fold increase in EC₅₀-ratio for HepG2/HT-29 (both relative to the respective mean hIL-22 values). EC₅₀-ratios for both cell lines are also above the upper values for the range of the corresponding hIL-22 EC₅₀ ratios.

**Table 7B: EC₅₀ of compound 1 (wild-type human IL-22 protein), and compound 3 (N94F modified IL-22 variant), in HT-29 (colon cell line), HaCaT (skin cell line) and HepG2 (liver cell line), *Data presented as geometric mean (range); NE - not estimated, because no meaningful EC50 value could be established due to the very low Eₘₐₓ.**

| **.Tested compounds** | | | **EC₅₀ ratios** | |
|---|---|---|---|---|
| **Compound ID** | **SEQ ID NO:** | **Modification relative to SEQ ID NO: 1** | **HaCaT/HT-29** | **HepG2/HT-29** |
| 1 | 1 | None | 8.3 (4.3-16.6)^{#} | 6.4 (3.6-10.3)^{#} |
| 3 | 3 | I19F | **21.5** | **27.4** |

### Conclusion

Compounds with this modification can be used to develop colon-selective variant drugs due to their potent effects in colon and none or very limited effects in skin and liver (with skin being the tissue which currently presents with the most unwanted effects in clinical trials of IL-22 variant drug candidates).

From example 3, it can be seen that the I19F modification represents an opportunity for developing a potential colon-selective modified IL-22 variant drug candidate.

This conclusion can be made based on the finding that compound 3 is an IL-22 variant with pharmaceutically relevant Eₘₐₓ values, as well as *wild-type relative Eₘₐₓ values* moderately favouring the colon over both the skin and liver. Together with the substantially increased potency in colon over both skin and liver, this modification is a promising one to apply in modified IL-22 variants for pharmaceutical applications.

### Example 4 - In vitro efficacy (Eₘₐₓ) and potency (EC₅₀) in the colon (HT-29), skin (HaCaT) and liver (HepG2) cell lines, of modified IL-22 variants comprising a N94F modification

Cell lines where cultures according to Assays I, II and III, respectively, depending in the cell line, the compounds as indicated were prepared for testing according to Assay IV and then used for the stimulation-step of the cultured cells as described in Assay V. The Quantification and Data analysis was performed according to Assays VI and VII.

### Results - IL-22 variants comprising a N94F modification are more efficacious and potent in colon than in skin and liver.

The below tables and corresponding Figure 7 show the results of testing of the indicated compounds 1 and 6 in the three cell lines, representing colon, skin and liver epithelia cells, respectively.

### Efficacy (Eₘₐₓ)

The Eₘₐₓ of each cell line after stimulation with the indicated compounds can be reviewed in Table 8A. Compound 6 presents with Eₘₐₓ values within the range of human IL-22 (compound 1), in colon (HT-29 cell line) and in skin (HaCaT-cell line), and slightly below the lower range in liver (HepG2 cell line).

**Table 8A: Eₘₐₓ (alpha-signal counts) of compound 1 (wild -type human IL-22 protein), and compound 6 (N94F modified IL-22 variant), in HT-29 (colon cell line), HaCaT (skin cell line) and HepG2 (liver cell line). #Data presented as a median (IQR).**

| **Tested compounds** | | | **Eₘₐₓ (alpha-signal counts)** | | |
|---|---|---|---|---|---|
| **Compound ID** | **SEQ ID NO:** | **Modification relative to SEQ ID NO: 1** | **in HT-29** | **in HaCaT** | **in HepG2** |
| 1 | 1 | None | 441 (303-504)^{#} | 453 (378-625)^{#} | 702 (519-892)^{#} |
| 6 | 6 | N94F | 418 | 476 | 426 |

### Wild-type human IL-22 protein (compound 1) relative Eₘₐₓ

As shown in Table 8B, the N94F substitution results in neutral *wild-type relative Eₘₐₓ values.*

**Table 8B: Relative Eₘₐₓ of compound 1 (wild-type human IL-22 protein), and compound 6 (N94F modified IL-22 variant), in HT-29 (colon cell line), HaCaT (skin cell line) and HepG2 (liver cell line).**

| **Tested compounds** | | | **hIL-22 relative Eₘₐₓ (%)** (Eₘₐₓ as percentage of corresponding wild-type human IL-22 protein (compound 1) value) | | |
|---|---|---|---|---|---|
| **Compound ID** | **SEQ ID NO:** | **Modification relative to SEQ ID NO: 1** | **in HT-29** | **in HaCaT** | **in HepG2** |
| 6 | 6 | N94F | 83 | 99 | 81 |

### Potency (EC₅₀)

Compound 6 (N94F modification) presents with EC₅₀ values below the lower normal range of the human IL-22 reference for EC₅₀ values for colon (HT-29 cell line) and within the higher end of the normal range in the other two tissues (HaCaT and HepG2 cell lines), as shown in Table 9A.

**Table 9A: EC₅₀ of compound 1 (wild-type human IL-22 protein), and compound 6 (N94F modified IL-22 variant), in HT-29 (colon cell line), HaCaT (skin cell line) and HepG2 (liver cell line), *Data presented as geometric mean (range); NE - not estimated, because no meaningful EC50 value could be established due to the very low Eₘₐₓ.**

| **Tested compounds** | | | **EC₅₀ (µM)** | | |
|---|---|---|---|---|---|
| **Compound ID** | **SEQ ID NO:** | **Modification relative to SEQ ID NO: 1** | **in HT-29** | **HaCaT** | **HepG2** |
| 1 | 1 | None | 1.8e-03 (6.9e-04 - 3.3e-03)* | 1.1e-02 (5.4e-03 - 2.1e-02)* | 1.6e-02 (6.8e-03 - 4.8e-02)* |
| 6 | 6 | N94F | 4.7E-04 | 4.2E-02 | 1.9E-02 |

### Potency (EC₅₀)- skin/colon and liver/colon - ratios

Table 9B shows that the tissue-relative EC₅₀-ratios for compound 6 (N94F modification) reveal a 10.8-fold increase in EC₅₀-ratio for HaCaT/HT-29 and 6.3-fold increase in EC₅₀-ratio for HepG2/HT-29 (both relative to the respective mean hIL-22 ratio values). EC₅₀-ratios for both cell lines are also above the upper values for the range of the corresponding hIL-22 EC₅₀ ratios.

**Table 9B: EC₅₀ of compound 1 (wild-type human IL-22 protein), and compound 6 (N94F modified IL-22 variant), in HT-29 (colon cell line), HaCaT (skin cell line) and HepG2 (liver cell line), *Data presented as geometric mean (range); NE - not estimated, because no meaningful EC₅₀ value could be established due to the very low Eₘₐₓ.**

| **Tested compounds** | | | **EC₅₀ ratios** | |
|---|---|---|---|---|
| **Compound ID** | **SEQ ID NO:** | **Modification relative to SEQ ID NO: 1** | **HaCaT/HT-29** | **HepG2/HT-29** |
| 1 | 1 | None | 8.3 (4.3-16.6)^{#} | 6.4 (3.6-10.3)^{#} |
| 6 | 6 | N94F | **89.7** | **40.3** |

### Conclusion

From example 4 it can be seen that the amino acid modification N94F represents an excellent opportunity for developing a potential colon-selective modified IL-22 variant drug candidate.

This conclusion is based on the finding that compound 6 (N94F modification) results in an IL-22 variant with pharmaceutically relevant Eₘₐₓ values and *wild-type relative Eₘₐₓ values.* Together with the substantial increase in potency in the colon cell line over both the skin and liver cell lines, this modification provides relevant characteristics to apply in modified IL-22 variants for pharmaceutical applications.

Compounds with this modification can be used to develop colon-selective variant drugs due to their potent effects in the colon and none or only limited effects in the skin and liver, skin being the tissue which currently present with the most unwanted effects in clinical trials of IL-22 variant drug candidates.

## Claims

1. An IL-22 variant having a sequence identity of at least 90% to SEQ ID NO:1, comprising an amino acid substitution relative to SEQ ID NO: 1 in at least one of positions 19, 87 or 94.

2. The variant according to claim 1, having the general formula (I): 
APISSHCRLD KSNFQQPY-X19-T NRTFMLAKEA SLADNNTDVR LIGEKLFHGV
SMSERCYLMK QVLNFTLEEV LFPQSDRFQP YMQEVV-X87-FL-A-RLS-X94-RLSTCH
IEGDDLHIQR NVQKLKDTVK KLGESGEIKA IGELDLLFMS LRNACI
general
formula (I)
 wherein
a. X₁₉ is I, which is the native amino acid of SEQ ID NO:1 in position 19 or is a non-native hydrophobic amino acid;
b. X₈₇ is P, which is the native amino acid of SEQ ID NO:1 in position 87 or a hydrophobic amino acid or a positively charged amino acid, suitably a hydrophobic amino acid comprising an aromatic ring; and/or
c. X₉₄ is N, which is the native amino acid substitution of SEQ ID NO:1 in position 94 or is a hydrophobic amino acid; and
wherein at least one of said amino acids X₁₉, X₈₇ and X₉₄ is a non-native amino acid relative to SEQ ID NO:1; and wherein said IL-22 variant further optionally comprises additional amino acid modifications, such as substitutions in other positions than X₁₉, X₈₇ or X₉₄, provided that the sequence identity of the IL-22 variant is between 94% and 99.5% to SEQ ID NO:1.

3. The IL-22 variant according to claim 2, wherein at least two of said amino acids X₁₉, X₈₇ and X₉₄ are non-native amino acids relative to SEQ ID NO:1.

4. The IL-22 variant according to any one of the preceding claims 2 or 3, comprising up to 7 additional amino acid modifications, such as substitutions in other positions than X₁₉, X₈₇ or X₉₄, provided that the total number of modifications relative to SEQ ID NO:1 is between 2 and 8, preferably between 2 and 6.

5. The IL-22 variant according to any one of the preceding claims 2 to 4, wherein at least one amino acid substitution is selected from:
a. X₁₉ is I, which is the native amino acid of SEQ ID NO:1 in position 19, or selected from the group consisting of: F, W, A, V and L;
b. X₈₇ is P, which is the native amino acid of SEQ ID NO:1 in position 87 or selected from the group consisting of: R, F, H, K, W, A, V, I and L; and/or
c. X₉₄ is N, which is the native amino acid substitution of SEQ ID NO:1 in position 94 or selected from the group consisting of: F, W, A, V, I, and L; and
wherein at least one of said amino acids X₁₉, X₈₇ and X₉₄ is a non-native amino acid relative to SEQ ID NO:1; further comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

6. The IL-22 variant according to any one of the preceding claims 2 to 5, wherein at least one amino acid substitution is selected from:
a. X₁₉ is I, which is the native amino acid of SEQ ID NO:1 in position 19, F or W;
b. X₈₇ is P, which is the native amino acid of SEQ ID NO:1 in position 87 or selected from the group consisting of: R, F, H, and W; and/or
c. X₉₄ is N, which is the native amino acid substitution of SEQ ID NO:1 in position, F or W; and
wherein at least one of said amino acids X₁₉, X₈₇ and X₉₄ is a non-native amino acid relative to SEQ ID NO:1; and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

7. The IL-22 variant according to any one of the preceding claims 2 to 6, wherein at least one amino acid substitution is selected from:
a. X₁₉ is I, which is the native amino acid of SEQ ID NO:1 in position 19, F or W;
b. X₈₇ is P, which is the native amino acid of SEQ ID NO:1 in position 87, R or F; and/or
c. X₉₄ is N, which is the native amino acid substitution of SEQ ID NO:1 in position, F or W; and
wherein at least one of said amino acids X₁₉, X₈₇ and X₉₄ is a non-native amino acid relative to SEQ ID NO:1; and further optionally comprising up to 7 additional amino acid modifications, such as substitutions in other positions than X₁₉, X₈₇ or X₉₄, provided that the sequence identity of the IL-22 variant is between 94% and 99.5% to SEQ ID NO:1.

8. An IL-22 variant according to any preceding claim 2-7, wherein said optional additional amino acid modifications are selected from
a. the group consisting of: I19F, P87R or N94F; and/or
b. the group consisting of: S12E, N13A, Q83A, E84A, R91A or R95A; and/or
c. the group consisting of: A1G, A1H, N35D, N35H, N35Q, N64D, N64Q, N64W, A90F, Q113R, and K114R; and/or
d. N35Q and N64Q.

9. An IL-22 variant according to any of the preceding claims, wherein the variant additionally comprises an N-terminal peptide and/or a C-terminal peptide extension.

10. A derivative of IL-22 comprising an IL-22 variant according to any preceding claim, and wherein the derivatization comprises one or more of (i) a covalently attached fatty acid, (ii) a covalently attached PEG group and (iii) formation of an Fc-fusion protein comprising the IL-22 variant and at least a portion of an antibody Fc protein.

11. A pharmaceutical composition comprising an IL-22 variant according to any of claims 1-9 or a derivative according to claim 10, and a pharmaceutically acceptable vehicle, wherein the pharmaceutical composition is suitable for administration by inhalation, administration by injection, oral administration, rectal administration or ocular administration, optionally wherein the administration by injection is intraperitoneal, intramuscular, subcutaneous or intravenous.

12. An IL-22 variant according to any of claims 1-9, a derivative according to claim 10 or a pharmaceutical composition according to claim 11, for use in therapy.

13. An IL-22 variant according to any of claims 1-9, a derivative according to claim 10or a pharmaceutical composition according to claim 11, for use in a method of treating a metabolic, pulmonary, gut, pancreatic, kidney, or central nervous system (CNS) disease, disorder and/or condition.

14. A variant, derivative or pharmaceutical composition for use according to claim 13, wherein:
(vii) the metabolic disease, disorder and/or condition is obesity, prediabetes, insulin resistance, diabetes type 1, diabetes type 2, metabolic dysfunction-associated steatohepatitis (MASH), hyperlipidaemia, hyperglycaemia or hyperinsulinemia;
(viii) the pulmonary disease, disorder and/or condition is chronic obstructive pulmonary disease (COPD), cystic fibrosis, bronchiectasis, idiopathic pulmonary fibrosis, acute respiratory distress syndrome, a chemical injury, a viral infection, a bacterial infection or a fungal infection;
(ix) the gut disease, disorder and/or condition is inflammatory bowel disease (IBD), ulcerative colitis, Crohn's disease, graft-versus-host-disease (GvHD), coeliac disease, a chemical injury, a viral infection or a bacterial infection;
(x) the pancreatic disease, disorder and/or condition is pancreatitis;
(xi) the kidney disease, disorder and/or condition is acute kidney disease or chronic kidney disease; or
(xii) the CNS disease, disorder and/or condition is multiple sclerosis.
